(19) Europäisches Patentamt / European Patent Office / Office européen des brevets

(11) **EP 4 312 582 B1**

(12) **EUROPEAN PATENT SPECIFICATION**

(45) Date of publication and mention of the grant of the patent:
**25.03.2026 Bulletin 2026/13**

(21) Application number: **22720321.3**

(22) Date of filing: **31.03.2022**

(51) International Patent Classification (IPC):
**A23K 10/00** (2016.01)    **G16H 20/60** (2018.01)
**G16H 10/40** (2018.01)

(52) Cooperative Patent Classification (CPC):
**G16H 50/20; A23K 20/24; A23K 20/26; A23K 50/75; G16H 10/40; G16H 20/60; G16H 50/30**

(86) International application number:
**PCT/EP2022/058513**

(87) International publication number:
**WO 2022/207767 (06.10.2022 Gazette 2022/40)**

(54) **MODEL-BASED DETECTION OF DEFICIENCY IN ANIMAL'S NUTRITIONAL STATE**

MODELLBASIERTE ERKENNUNG VON MÄNGELN IM ERNÄHRUNGSZUSTAND VON TIEREN

DÉTECTION DES CARENCES DANS LÉTAT NUTRITIONNEL DES ANIMAUX BASÉE SUR UN MODÈLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.03.2021 US 202163168472 P**
**31.03.2021 US 202163168477 P**
**31.03.2021 US 202163168492 P**
**31.03.2021 US 202163168494 P**
**06.04.2021 EP 21167007**

(43) Date of publication of application:
**07.02.2024 Bulletin 2024/06**

(73) Proprietor: **DSM IP Assets B.V.**
**6221 BE Maastricht (NL)**

(72) Inventors:
• **COWIESON, Aaron**
**4303 Kaiseraugst (CH)**
• **FROST, Thomas**
**4303 Kaiseraugst (CH)**
• **LAPRADE, Lisa, Ann**
**4303 Kaiseraugst (CH)**
• **PHILLIPS, Chelsea**
**4303 Kaiseraugst (CH)**

(74) Representative: **DSM IP ANH**
**DSM Nutritional Products AG**
**Wurmisweg 576**
**4303 Kaiseraugst (CH)**

(56) References cited:
WO-A1-2020/109348    WO-A2-2021/074332
US-A1- 2005 126 500    US-A1- 2007 212 713
US-A1- 2015 169 847    US-A1- 2016 012 748

• MICHAEL P. MARTIN ET AL: "Selected Blood Chemistry and Gas Reference Ranges for Broiler Breeders Using the i-STAT Handheld Clinical Analyzer", AVIAN DISEASES., vol. 54, no. 3, 1 September 2010 (2010-09-01), US, pages 1016 - 1020, XP055682766, ISSN: 0005-2086, DOI: 10.1637/9223-122209-Reg.1
• PIETRO CELI ET AL: "Biomarkers of gastrointestinal functionality in animal nutrition and health", ANIMAL FEED SCIENCE AND TECHNOLOGY, vol. 250, 1 April 2019 (2019-04-01), AMSTERDAM, NL, pages 9 - 31, XP055683695, ISSN: 0377-8401, DOI: 10.1016/ j.anifeedsci.2018.07.012

- **"Diseases of Poultry, 13th Edition", 4 October 2013, JOHN WILEY & SONS, INC, article STEPHEN R COLLETT: "Chapter 1 Principles of Disease Prevention, Diagnosis, and Control", pages: 3 - 60, XP055683856**
- **MAZDA JENAB ET AL: "Biomarkers in nutritional epidemiology: applications, needs and new horizons", HUMAN GENETICS, SPRINGER, BERLIN, DE, vol. 125, no. 5-6, 9 April 2009 (2009-04-09), pages 507 - 525, XP019716454, ISSN: 1432-1203**

**Description**

**FIELD OF THE DISCLOSURE**

The present disclosure generally relates to precision animal nutrition. In particular, the present disclosure relates to a system and computer-implemented method for detecting a deficiency in a nutritional state of at least one animal. The disclosure further relates to a computer-readable medium comprising data representing instructions arranged to cause a processor system to perform the computer-implemented method.

**BACKGROUND ART**

Animal nutrition focuses on dietary nutrients needs of animals. Carbohydrates and proteins are well-known nutrients. Important classes of micronutrients are minerals and vitamins. Micronutrients are required in animal diets for health and welfare. Therefore, they are essential for the agricultural production of milk, meat, fiber, and eggs. Profitability of livestock or poultry production systems can be increased through optimized nutritional management. If nutrients are fed in a concentration higher than needed, resources and money are wasted. On the other hand, if not enough nutrients are fed, animals may underperform: they do not gain weight fast enough, give less milk or lay less eggs.
There are several possible reasons why animals may be fed with too much or too little of one or multiple (micro-) nutrients:

*Nutrient concentration in feed varies*

Variation in ingredient quality and nutrient concentration have a large impact on the utilization of nutrients by the animal. Factors that influence ingredient quality and nutrient variation can be harvest year and environmental conditions (US Grains Council, Corn Harvest Quality Report 2019/2020. Washington, DC), genetic variety (Knudsen, K.E.B., 2014. Fiber and nonstarch polysaccharide content and variation in common crops used in broiler diets. Poult. Sci. 93, 2380-2393), post harvest processing (Odjo, S. et al., 2012. Influence of drying and hydrothermal treatment of corn on the denaturation of salt-soluble proteins and color parameters. J. Food Engineering. 109, 561-570) and storage conditions (Yin, D., Yuan et al., 2017. Effect of storage time on the characteristics of corn and efficiency of its utilization in broiler chickens. Anim. Nutr. 3, 252-257).

*Feed additives are not homogenously distributed in the feed*

The amount of a micronutrient that is present in crop or any other feed ingredient may be increased by adding a feed additive to the feed. The concentration of feed additives in feed is so small that it is often indicated in ppm (parts per million). Homogeneously distributing a very small amount of feed additive within a large amount of feed is challenging.

*Bioavailability of nutrients*

Intestinal malabsorption (e.g., due to diarrhea) may have a negative impact on oral bioavailability of a swallowed nutrient. An increased nutrient intake may help to compensate for impaired intestinal absorption. There are other scenarios where low bioavailability can be compensated by an increased oral intake.
Deficiencies in the feed, such as varying quantities of nutrients and non-homogenous distribution in the feed, may be determined by wet chemistry methods However, wet chemistry methods have downsides, in particular for a farmer seeking to determine the nutritional state of his/her animals, as they involve harsh chemicals, are time consuming/expensive and require specialized lab equipment/trained personnel. In addition, wet chemistry methods do not take into account the possible low bioavailability of the nutrients, e.g., due to malabsorption or due to other factors.
There is a need to detect a deficiency in the nutritional state of animals in an improved manner, preferably one which is more accessible to farmers.
Furthermore, if an issue has been detected, feed cannot easily be modified. Due to cost reason, feed is prepared in large batches. If the composition of a batch proves to be inadequate, the prepared batch of feed must first be used up. Modifying the composition of feed has proven to be a very slow response to a detected feed issue. There is a need to improve nutritional management of livestock production systems.
Heat stress substantially reduces the growth rate of broiler chicks and other livestock species. One reason is respiratory alkalosis (i.e. high blood pH). This goes along with lower than normal K+ blood concentration and lower than normal Na+ concentration. Therefore, Deyhim et al. suggest to supplement broilers' drinking water with KCl or NaCl (Deyhim et al., "Effect of heat stress and drinking water salt supplements on plasma electrolytes and aldosterone concentration in broiler chickens", Int J Biometeorol. 1995 May;38(4):216-7. doi: 10.1007/BF01245392).

Unfortunately, supplementing KCl or NaCl comes with a concomitant rise in chloride and thus, raises the risk of hyperchloremia (i.e. too much chloride in the blood). Hyperchloremia could be prevented by adding an alternative salt of potassium or sodium to broilers' drinking water. Teeter et al. suggest to control heat stress by supplementation with alternative electrolyte salts such as sodium bicarbonate (Teeter R.G., Smith M.O. and Owens F.N. (2000), "Chronic heat stress and respiratory alkalosis: occurrence and treatment in broiler chicks", Pp. 160-169 in Proc. Georgia Nutr. Conf. for the Feed Indus. Univ. Georgia, Atlanta).

Supplementing sodium bicarbonate and potassium bicarbonate does not come without any risk either. Hayat et al. have found that "sodium bicarbonate and potassium bicarbonate supplements for broilers can cause poor performance at high temperatures" (J. Hayat, D. Balnave & J. Brake, (1999), British Poultry Science, 40:3, 411-418, DOI: 10.1080/00071669987539). The negative impact of these supplementations depends on the route of administration. In Hayat's study, supplements of NaHCO3 were more toxic to broiler chickens when supplied in the drinking water than in the diet.

High temperature remains a major limiting factor in livestock animal production. The problem becomes even more severe when high temperature is accompanied by high humidity. There is also a need for an improved strategy to combat heat stress.

Prior art document US 2008/234995 concerns a method to modify inputs of an animal production system to produce output optimized according to certain characteristics. Prior art document US 2016/012748 A1 discloses a method for the provision of recommendations on improved nutrition, health and/or wellness protocols for an animal. Prior art document WO 2020/109348 A1 discloses a method to adjust supply values based on animal status data, farm condition data and sensor data.

## SUMMARY

The invention is restricted to the claims. Any aspect of the disclosure or embodiment which does not fall under the scope of the claims does not pertain to the invention.

### 1. Model-Based Detection of Deficiency in Animal's Nutritional State

A first aspect of the disclosure provides a system for detecting a deficiency in a nutritional state of at least one animal. A further aspect of the disclosure provides a computer-implemented method for detecting a deficiency in a nutritional state of at least one animal. A further aspect of the disclosure provides a computer-readable medium comprising data to cause a processor system to perform the method.

The above measures provide a system and method for detecting a deficiency in a nutritional state of at least one animal. Surprisingly, the system and method use ex-vivo blood measurements to determine the nutritional state of the animal(s). While such blood measurements may appear complex and not easily accessible to farmers, e.g., by requiring farmers to send blood samples to a lab, the advent of point-of-care devices has enabled ex-vivo blood measurements to be performed *in situ,* meaning within the agricultural setting in which the animals are raised, e.g., in the farm or field. Accordingly, a farmer may easily perform and obtain the result of an ex-vivo blood measurement. However, such blood measurement data may be not, or at least not easily, interpretable by a farmer, and in particular, not easily relatable to a deficiency in the feed or in the bioavailability of nutrients.

To address this problem, a computer system is provided which may be configured to determine, from the blood measurement data, a measured concentration of a biomarker of a feed nutrient in the feed of the animal. Such a biomarker may for example be a compound found in the blood whose concentration in the blood correlates with the ingested amount of the feed nutrient. In addition, a nominal concentration of the feed nutrient in the feed of the at least one animal is obtained. Such a nominal concentration may represent an expected concentration of the feed nutrient, which may for example be obtained based on the feed recipe of the feed which is fed to the at least one animal. The computer system may detect a deficiency in the nutritional state of the at least one animal by checking if the measured biomarker concentration is in agreement with the nominal feed nutrient concentration. Surprisingly, while both quantities relate to different domains, e.g., blood vs. feed, they may nevertheless be compared by computer-based modelling.

In particular, a parameterized model is provided which has been generated, e.g., by machine learning, to model a relation between concentrations of the feed nutrient in feed of animals and concentrations of the biomarker in the blood of at least some of the animals. For example, the parameterized model may be generated using regression analysis of experimental data obtained from a controlled setting, where feed with known nutrient concentrations is fed to animals while the biomarker concentrations in blood of some animals are measured, for example using point-of-care devices or wet chemistry methods. While the generation of such type of parameterized model may be less accessible to a farmer, e.g., by requiring model fitting capabilities, the resulting parameterized model may be made available in a manner which may be easily accessible to the farmer, namely via the aforementioned computer system, being for example a local or web-accessible system.

The agreement between the measured biomarker concentration and the nominal feed nutrient concentration may be determined by either estimating a biomarker concentration from the nominal feed nutrient concentration, namely by inputting the nominal feed nutrient concentration into the parameterized model to obtain an estimated biomarker concentration, or by estimating a feed nutrient concentration from the measured biomarker concentration, namely by inputting the measured biomarker concentration into the parameterized model to obtain an estimated feed nutrient concentration. Either way, an estimated value may be obtained which may be compared to the 'reference' value, being either the measured biomarker concentration in the blood domain or the nominal feed nutrient concentration in the feed domain. If both values are in agreement, there may be a low probability of a deficiency in the nutritional state of the at least one animal as the feed provided to the animals indeed results in a biomarker concentration in the blood which is predicted by the parameterized model. However, in case of a disagreement, there may have a higher probability of a deficiency, which probability may increase with an increase in the difference between the estimated and reference value. Namely, such disagreement may indicate a deficiency in the feed, in that the actual concentration of the feed nutrient in the feed may deviate from the expected nominal concentration, and/or that there is insufficient bioavailability of the feed nutrient in the blood, in that the nominal feed nutrient concentration should have resulted in a higher or lower biomarker concentration in the blood of the at least one animal.

Accordingly, output data may be generated as a function of the difference, e.g., as a function of the presence and/or the amount of disagreement, which output may be used to address the nutritional deficiency. For example, the output data may represent a recommendation to a user, e.g., the farmer, to check the actual concentration of the feed nutrient in the feed, or to check for signs of malabsorption, etc. In case the farmer is recommended to check the actual concentration of the feed nutrient in the feed, the aforementioned wet chemistry methods may be used, but such use may be limited to cases where the estimated nutrient concentration (significantly) deviates from the expected nutrient concentration. In case of any recommendation, the recommendation may be stored at least in part as a text which may be output where and when appropriate. In other examples, the output data may represent control data to an actuator of a dispensing system, by which control data the dispensing system may be controlled to dispense nutritional supplements, e.g., additives, to supplement the feed nutrient in the feed. In yet other examples, the output data may simply contain a numerical value of the difference. Various other types of output data and uses of the output data are equally conceived but have in common that they allow control the livestock or poultry production process to address the deficiency in the nutritional state of the at least one animal.

In some embodiments, the processor subsystem may be configured to threshold the difference, e.g., by an absolute or relative threshold, and generate the output data conditionally or differently depending on if the threshold is exceeded. This allows different behavior for significant and insignificant disagreements.

In some embodiments, the nominal concentration may be obtained from a nutritional log which may characterize the feed fed to the at least one animal. For example, a database may be used to relate a recipe of the feed to concentrations of feed nutrients. This may avoid a need for a user, such as the farmer, to manually determine and/or enter the nominal concentration into the computer system.

In some embodiments, the output data may be, or may comprise, control data for an actuator which may be used to adjust a composition of nutritional supplements and/or to dispense the nutritional supplements. As such, the computer system may function as a feedback control system which may, based on the difference of the estimated nutrient concentration to the nominal nutrient concentration or based on the difference of the measured biomarker concentration to the estimated biomarker concentration, provide supplements to the animals. It is noted that if the difference is evaluated in the biomarker domain, the parameterized model may be used to determine which amount of feed nutrient compensates for said difference. The nutritional supplements may also be referred to as 'additives' when added to the feed or drinking water. The actuator may for example be an electric, hydraulic, pneumatic, thermal, magnetic and/or mechanical actuator, or a combination of such actuators. Specific yet non-limiting examples include electrical motors, hydraulic cylinders, pneumatic actuators, servomechanisms, solenoids, stepper motors, etc. The actuator may be part of a dispensing system; by controlling the actuator, the dispensing system may be controlled by the computer system. For example, the actuator may control a valve of a medicator or other metering device.

In some embodiments, the nutritional supplements may be dispensed via drinking water. For example, a medicator or other type of metering device may be provided and controlled by the computer system to directly dispense the nutrients into the drinking water, or to dispense an aqueous solution of the nutrients into the drinking water, e.g., via a valve connected to a water line. This may allow easier supplementation of nutrients than via the feed. Namely, due to cost reason, feed is typically prepared in large batches. If the composition of a batch proves to be inadequate, the prepared batch of feed must be used up first. Modifying the composition of feed has proven to be a very slow response to a detected feed issue. In contrast, an extra amount of the nutrient may be easily added to the drinking water. This may be quicker than modifying the animal's feed. Additionally, nutrients may be added to the animal's drinking water at any moment in time, even if there is a large left over of possibly inadequate feed that has been prepared beforehand.

In some embodiments, environmental parameters may be taken into account when dispensing nutritional supplements. For example, the average water consumption of animals rises when the temperature rises. The computer system may take this relationship into account by receiving temperature data, e.g., from a sensor located on-premises or from a weather forecast service, and by controlling the dispensing of the nutritional supplements based on the temperature data. For example, if it is expected that the animals increase their water consumption, some nutritional supplements may be dispensed in a reduced quantity per volume unit of water (e.g., per hectoliter) to avoid over-supplementation. In some embodiments, the quantity and/or the type of the nutritional supplements may be adjusted based on the temperature data. For example, if it is expected that the animals experience or will experience heat stress, (more) electrolytes may be dispensed via the drinking water.

In some embodiments, the biomarker identified in the blood measurement data relate to a presence of Na+, K+ and/or Cl- in the blood, and the parameterized model may be used to estimate the amount of Na+, K+ and/or Cl- to be supplemented, for example via drinking water, given the concentration of Na+, K+ and/or Cl- in the blood. The animals may be fed feed that comprises a relatively high amount of potassium and sodium and a relatively low amount of chloride. Such feed has a comparatively low dietary electrolyte balance (dEB). The feed's comparatively low dietary electrolyte balance (dEB) may be compensated by adding KCl and/or NaCl to the drinking water of the fed animals. This may be done without any substantial risk for hyperchloremia because feed having a low dietary electrolyte balance (dEB) comprises relatively little chloride. Adding electrolytes (such as NaCl or KCl) to the drinking water allows for a very short reaction time, whereas altering dEB in feed takes several weeks to filter through to the animals, drinking water solutions can have an effect within hours. Metering the correct amount of an electrolyte (such as KCl and NaCl) to animals' drinking water may be extremely challenging, and it is even more challenging when animals are under heat stress because heat stress tends to increase water consumption. The more supplemented water an animal consumes, the higher the ingested dose of the supplementation is. Furthermore, the increased water consumption might result in a reduced blood concentration of, e.g., Na+ and/or K+ (hemodilution). In some embodiments, the appropriate amount of Na+, K+ and/or Cl- to be metered into animal's drinking water may be determined by measuring ex vivo the animal's blood concentration of Na+, K+ and/or Cl-, using a suitable point-of-care device. The measured blood concentrations may be the input of the parameterized model whose output allows to determine the amount of Na+, K+ and/or Cl- to be metered into the water flow line. For metering, a medicator may for example be used. Relying on measured blood concentrations of Na+, K+ and/or Cl- has the following benefits: (a) metering electrolytes into animal's drinking water on the basis of the usual paper exercise is rarely successful because water intake and ambient temperature can vary (b) the usual paper exercise of a nutritionist does not take into account natural variance in the K+, Na+ and Cl- concentration in the dietary ingredients.

In some embodiments, the nutrient concentration may be estimated by the parameterized model based on the average age of the animals, or on an individual age of an animal. This may account for the fact that the relation between nutrients in feed and the concentration of biomarkers in the blood may be age dependent. Accordingly, the parameterized model may receive the age as additional input parameter to provide an age-dependent estimate of the nutrient concentration.

In some embodiments, a method is conceived which may be part computer-implemented and part not computer-implemented. For example, the method may comprise a non-computer implemented step of using a point-of-care device to obtain an ex-vivo measurement of a sample of blood of at least one animal, while additionally comprising all or a number of steps of a computer-implemented method described in this specification. In another example, the method may additionally comprise a non-computer implemented step of feeding the animals with feed that has a low dietary electrolyte balance (dEB), and a computer-implemented step of controlling an actuator to dispense Na+, K+ and/or Cl- into the drinking water of the animals to compensate for a deficiency in Na+, K+ and/or Cl-. In some embodiments, a use of a point-of-care device to obtain an ex-vivo measurement of a sample of blood of at least one animal is conceived.

In some embodiments, a system is provided which comprises the computer system and at least one point-of-care device. The computer system may be located on premise, e.g., on a farm, but may in other embodiments also be a remote computer system, e.g., hosted on a cloud service, which may for example be accessible via a web interface via which a farmer may upload blood measurement data and receive feedback. In some embodiments, the computer system may directly communicate with the point-of-care device or with a data management system of the point-of-care device, for example via an API of the computer system and/or an API of the point-of-care device and/or an API of the point-of-care data management system.

## 2. Use of Blood Biomarkers to *Confirm Accuracy of Dietary Nutrient Supply to Production Animals*

The problems underlying a further aspect of the present disclosure are solved by measuring the response of blood biomarkers to variations in nutrient concentration in feed. Response-based nutrient recommendations are given to increase animal performance, lower feed cost and/or to lower analytical costs.

Measuring the response of blood biomarkers is more comprehensive than standard assays because this delivers information not only on total nutrient concentration of feed (which can be assessed in a laboratory) but also the bioavailability of these nutrients at the animal level.

Nutritional management of livestock production systems are optimized by rendering standard assays superfluous and/or by augmenting the informative value of standard assays.

### 3. Improving Accuracy of Electrolyte Supply to Production Animals

The solution of the problem underlying a further aspect of the present disclosure is preferably based on feed that comprises a relatively high amount of potassium and sodium and a relatively low amount of chloride. Such feed has a comparatively low dietary electrolyte balance (dEB).

Dietary electrolyte balance of a given feed is ((mg/kg Na+/23)+(mg/kg K+/38.1))-(mg/kg Cl-/35.5) and is usually reported in milliequivalents (mEq). If some of the feed's NaCl is replaced by sodium bicarbonate ($NaHCO_3$) or other alternative salts, the calculated mEq decreases. Nowadays, most feed comprise added sodium chloride (NaCl).

In a preferred embodiment of the disclosure, feed having a comparatively low dEB is prepared. Preferably, the feed's comparatively low dietary electrolyte balance (dEB) is then preferably compensated by adding KCl and/or NaCl to the drinking water of the fed animals. This can be done without any substantial risk for hyperchloremia because feed having a low dietary electrolyte balance (dEB) comprises relatively little chloride.

Furthermore, adding electrolytes (such as NaCl or KCl) to the drinking water allows for a very short reaction time: whereas altering dEB in feed takes several weeks to filter through to the animals, drinking water solutions can have an effect within hours.

Metering the correct amount of an electrolyte (such as KCl and NaCl) to animals' drinking water is extremely challenging. It is even more challenging when animals are under heat stress because heat stress tends to increase water consumption. The more supplemented water an animal consumes, the higher the ingested dose of the supplementation is. Furthermore, the increased water consumption might result in a reduced blood concentration of e.g. Na+ and/or K+ (hemodilution).

According to a preferred embodiment of the present disclosure, the correct amount of Na+, K+ and/or Cl- to be metered into animal's drinking water is determined by measuring *ex vivo* the animal's blood concentration of Na+, K+ and/or Cl-, using a suitable point-of-care device. The measured blood concentrations are the input of a model whose output allows to determine the amount of Na+, K+ and/or Cl- to be metered into the water flow line. For metering, a medicator is preferably be used. Relying on measured blood concentrations of Na+, K+ and/or Cl- has the following benefits:

(a) metering electrolytes into animal's drinking water on the basis of the usually paper exercise is rarely successful because water intake and ambient temperature can vary

(b) the usually paper exercise of a nutritionist does not take into account natural variance in the K+, Na+ and Cl- concentration in the dietary ingredients

### 4. Use of Drinking Water as a Medium to Supply Nutrients in an Amount That Precisely Meets the Nutritional Requirements of Animals to be Raised

A further aspect of the disclosure relates to feed surveillance done by methods other than wet chemistry. If the herein disclosed feed surveillance indicates that feed contains the expected nutrients in the expected amount ('adequate feed'), there is no need for any further action. This saves time and money.

If adequate feed is ingested by healthy animals in the normal amount, the animals' blood nutrient concentrations will be within the expected ranges. On the other hand, if a measured blood concentration of a nutrient is below the expected range, action needs to be taken.

In a less preferred embodiment of the disclosure, steps are taken to reveal the reason for the too low blood nutrient concentration. One possible reason is a lower than expected amount of the ingredient in the prepared feed. Another possible reason is a lower than normal bioavailability (e.g. due to animals suffering from diarrhea).

In a preferred embodiment of the disclosure, the reason for the too low blood nutrient concentration is not investigated. Instead, a more pragmatic approach is taken by giving the animal access to a higher amount of the missing nutrient. Eventually, the higher amount of the nutrient will bring the respective blood nutrient concentration back to normal.

In a preferred embodiment of the disclosure, an extra amount of the nutrient is added to the drinking water of the animal. This is quicker than modifying the animal's feed. Nutrients can be added to the animal's drinking water at any moment in time - even if there is a large left-over of possibly inadequate feed that has been prepared beforehand.

The problems underlying the present disclosure are preferably solved by a computer-implemented method of raising a flock of animals, said method comprising the steps:

(i) receiving a blood nutrient concentration of an animal of the flock, wherein said blood nutrient concentration has been measured *ex vivo,* and further receiving the nutritional log of the animal whose blood has been examined *ex vivo,*

(ii) providing a model which defines blood nutrient concentrations that are in range if the animal whose blood is examined *ex vivo* has been fed for a predetermined period of time with a predetermined amount of feed, said feed comprising the corresponding nutrient in a given concentration,

(iii) using the model of step (ii) to determine if the blood nutrient concentration received in step (i) is in range, below range or above range, and

(iv) if step (iii) reveals that the blood nutrient concentration received in step (i) is below range: recommending the addition of the nutrient to the drinking water of the flock of animals and/or adding the nutrient to the drinking water of the flock of animals.

To practice the herein disclosed method, the set-up of the present information disclosure can be used. Preferably, the set-up of the disclosure comprises:

a) at least one computer system,
b) at least one point-of-care device, and
c) at least one water flow line and at least one apparatus for metering nutrients into said water flow line, and

wherein said water flow line is suitable for watering animals.

The present disclosure also relates to the use of drinking water as a medium to supply nutrients to a flock of animals, wherein at least one nutrient is added to the drinking of the flock of animals, and wherein the amount of said at least one nutrient that is added to said drinking water is adapted to the nutritional requirements of the animals of said flock. An alternative embodiment of the disclosure relates to the use of least one blood concentration of a nutrient for metering a nutrient into drinking water of a flock of animals, wherein said blood concentration has been measured *ex vivo* in the blood of an animal of the flock. A higher amount of the nutrient is metered into the drinking water if the blood concentration of a nutrient is below a pre-determined range.

If the composition of a batch of feed proves to be inadequate (as indicated by a blood nutrient concentration that is below a pre-determined range), modifying the composition of the drinking water is a fast, easy and cost-effective manner to intervene - even if the prepared batch of feed has not yet been used up.

It will be appreciated by those skilled in the art that two or more of the above-mentioned embodiments, implementations, aspects and/or optional aspects of the disclosure may be combined in any way deemed useful.

Modifications and variations of the system, the computer-implemented method and/or the computer program product, which correspond to the described modifications and variations of another one of said entities, can be carried out by a person skilled in the art on the basis of the present description.

## BRIEF DESCRIPTION OF THE DRAWINGS

These and other aspects of the disclosure are apparent from and will be elucidated with reference to the embodiments described hereinafter. In the drawings,

Fig. 1 shows a computer system for detecting a deficiency in a nutritional state of at least one animal, comprising an input interface to a point-of-care device and an output interface to an actuator for controlling a dispensing system;

Fig. 2 shows the computer system on premise in a farm and being configured to wirelessly receive blood measurement data from a point-of-care device and to control an actuator to dispense nutrients into drinking water of the animals;

Figs. 3A and 3B show a client/server implementation of the computer system in which calculations are performed by a server and a client is used to upload blood measurement data and to display feedback calculated by the server;

Figs. 4A-4D show experimental data;

Fig. 5 shows a flowchart of steps of a computer-implemented method for detecting a deficiency in a nutritional state of at least one animal;

Fig. 6 shows a flowchart of steps of a computer-implemented method for generating a parameterized model for detecting a nutritional deficiency in animals;

Fig. 7 shows a computer-readable medium comprising data;

Fig. 8 shows an example flowchart of a computer-implemented method for confirming accuracy of dietary nutrient supply to production animals; and

Fig. 9 shows another example flowchart of a computer-implemented method for confirming accuracy of dietary nutrient supply to production animals.

## List of reference numbers

[0052] The following list of reference numbers is provided for facilitating the interpretation of the drawings and shall not be construed as limiting the claims.

| 20  | point-of-care device |
| 22  | blood measurement data |
| 40  | temperature sensor |
| 42  | temperature data |
| 60  | actuator |
| 80  | display |

| 100 | computer system |
| 120 | input interface subsystem |
| 140 | data storage |
| 142 | data representation of parameterized model |
| 160 | processor subsystem |
| 180 | actuator interface |
| 182 | actuator control data |
| 190 | display output |
| 192 | display data |

| 200 | farm building |
| 210 | water line |
| 220 | dispensing system |
| 230 | metering device |
| 240 | nutrient reservoir |
| 250 | drinking water dispenser |

| 300 | server |
| 320 | network |
| 340 | client device |
| 350 | display |
| 400 | method for detecting nutritional deficiency |
| 410 | access blood measurement data |
| 420 | determine biomarker concentration |
| 430 | provide parameterized model |
| 440 | obtain nominal concentration of feed nutrient |
| 450 | detect nutritional deficiency using parameterized model |
| 460 | generate output data |

| 500 | method for generating parameterized model |
| 510 | provide parameterized model |
| 520 | provide blood measurement data |
| 530 | provide nutrient concentration data |
| 540 | model data fitting |
| 550 | adapt parameters & output model |

| 600 | computer-readable medium |
| 610 | non-transitory data |

It should be noted that items which have the same reference numbers in different figures, have the same structural features and the same functions, or are the same signals. Where the function and/or structure of such an item has been explained, there is no necessity for repeated explanation thereof in the detailed description.

## DETAILED DESCRIPTION OF EMBODIMENTS

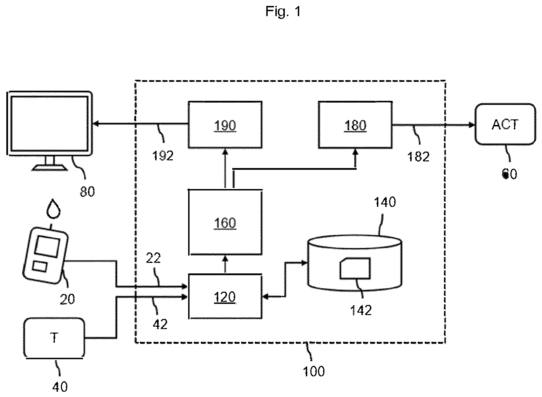

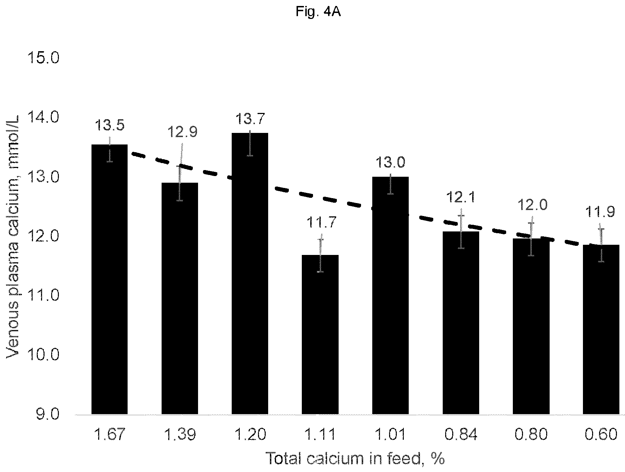

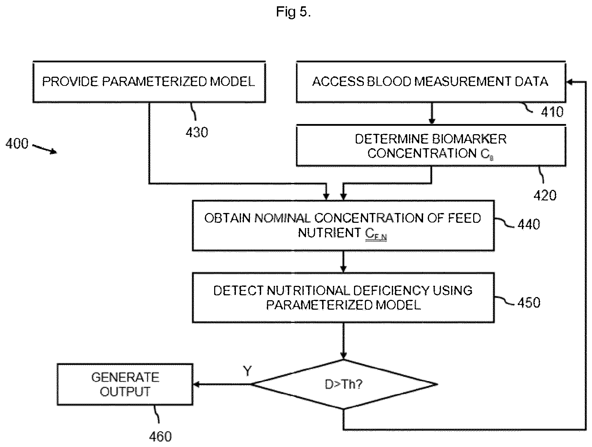

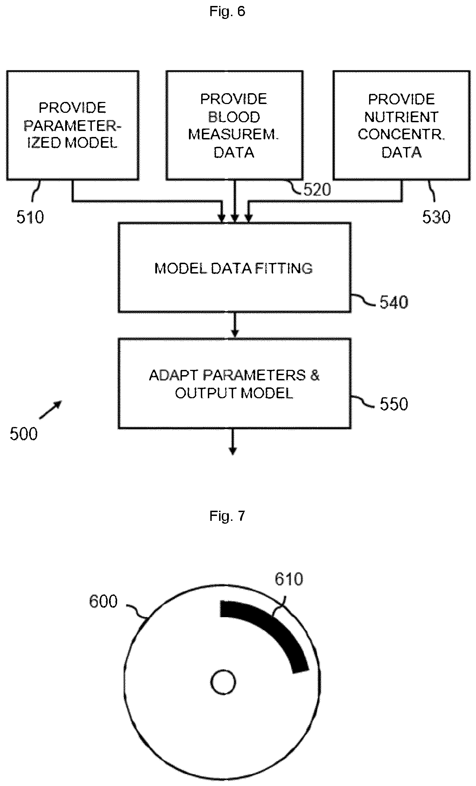

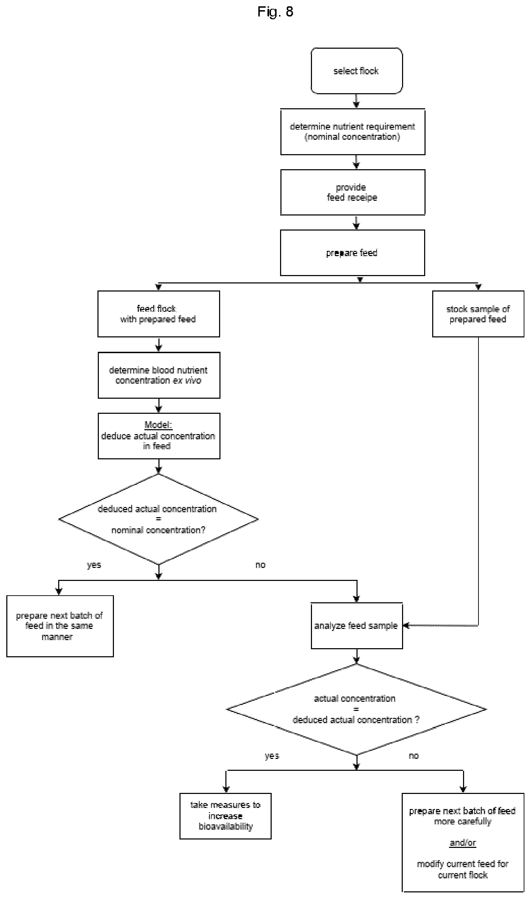

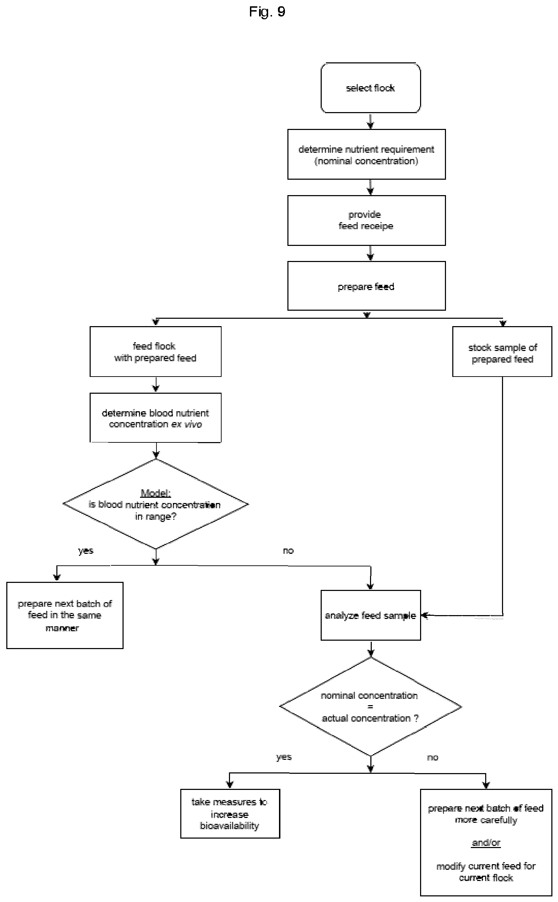

The following firstly introduces definitions used in this specification, then describes with reference to Figs. 1-3B various aspects of a system for detecting a deficiency in a nutritional state of animals, followed by a more detailed description how to detect such deficiencies, including various applications thereof (feed surveillance, raising animals, addressing heat stress in animals) and with reference to Figs. 4A-4D experimental data. Furthermore, with reference to Fig. 5, a computer-implemented method is described for detecting a deficiency in a nutritional state of animals, with reference to Fig. 6 a computer-implemented method for generating the parameterized model, and with reference to Fig. 7 a computer-readable medium comprising non-transitory data and used in some of the embodiments. Fig. 8 describes an example flowchart of a computer-implemented method for confirming accuracy of dietary nutrient supply to production animals. Fig. 9 describes another example flowchart of a computer-implemented method for confirming accuracy of dietary nutrient supply to production animals.

### *Definitions*

The following definitions are used in the context of the present disclosure.

- An "animal" is preferably a monogastric animal, preferably a bird, even more preferably a chicken or a turkey, or an "animal" is preferably a ruminant. Preferred chicken are boilers and laying hens. Most preferably, the animal is a broiler. A broiler is a chicken that is bred and raised specifically for meat production.
- A "nutrient" is a compound used by an animal to survive, grow and/or reproduce. Preferred nutrients are micronutrients. The notation "(micro-)nutrient" is to be read as "nutrient, wherein said nutrient is preferably a micronutrient".
- A "micronutrient" is a compound required in a small amount for the normal growth and development of an animal. Amounts of micronutrients in feed may be indicated in ppm, in mg per kg feed (e.g., vitamins) or in weight-%, based on the total weight of the feed (e.g., Ca2+, phosphate, Na+, Cl-, K+). Ca2+ is usually the highest content, preferably at <1.2 weight-% for broilers, <2 weight-% for turkeys, and <4 weight-% for laying hens, based on the total weight of the respective feed. In the context of the present disclosure, the term micronutrient refers preferably to a vitamin, a mineral (e.g., NaCl) or to an ion of a mineral (e.g., Na+). Proteins, fats, fibers, and carbohydrates are typically not considered as micronutrients.
- Point-of-care testing refers to diagnostic testing of a sample at or near the place where the animals are raised (e.g., at or near the chicken farm). A "point-of-care device" is a device that is suitable for performing point-of-care testing, preferably suitable for measuring ex vivo the concentration of a biomarker in a blood sample.
- A "biomarker" or "blood biomarker" is a compound found in the blood whose concentration in the blood correlates with the ingested amount of a given (micro-)nutrient. A biomarker may be the (micro-)nutrient itself, may be part of the (micro-)nutrient (e.g., an ion of a salt), may be a metabolite of the (micro-)nutrient or may be any other compound being indicative for the ingested amount of the (micro-)nutrient. For some (micro-)nutrients, blood concentrations of the corresponding blood biomarker are capped due to post absorptive changes, hormonal and/or transient responses. Whereas this can be taken into account by the herein described models, such (micro-)nutrients are less preferred.
- The expression "blood nutrient concentration" refers to the concentration of a (micro-)nutrient in the blood of an animal, e.g., indicated as mmol per liter blood. Blood nutrient concentrations are preferably measured ex vivo in a blood sample, preferably using a point-of-care device as herein described. In the context of the expression "blood nutrient concentration", nutrient is to be understood in a broad manner, referring to the (micro-)nutrient itself, to a metabolite thereof and to any other blood biomarker as herein defined. The biomarker may thus represent a blood nutrient. As such, the terms "blood nutrient concentration" and "biomarker concentration" may or may not be used synonymously in this specification.
- "Feed" is food suitable to be given to animals. The terms feed and animal feed are used synonymously. The source of the feed's (micro-)nutrients are feed ingredients. When feeding domesticated livestock or poultry, feed is most often a mixture of various feed ingredients. Exemplary feed ingredients are grains, milling by-products, fats, oils, and feed additives. Preferred feed additives are micronutrients such as vitamins and minerals. Feed additives are added to the other feed ingredients in a predetermined concentration.
- The expression "feed nutrient concentration" is to be understood as "feed (micro-)nutrient concentration" (e.g., it is preferably the concentration of a micronutrient). The expression refers to the concentration of a (micro-)nutrient in feed (e.g., indicated in weight-%, based on the total weight of the feed). A feed nutrient concentration can be a nominal concentration, an actual concentration, or a deduced concentration.
- A nominal concentration of a (micro-)nutrient is typically not based on a measured value. A "nominal concentration" is rather a target concentration that a nutritionist seeks to achieve (e.g., by providing a suitable feed recipe) or expects. If the target concentration is missed, the actual concentration is higher or lower than the nominal concentration. Thus, in reality, the concentration of a (micro-)nutrient in feed may or may not be the same as the (micro-)nutrient's nominal concentration.
- The reality is reflected by the actual concentration of the (micro-)nutrient. The value of an "actual concentration" is preferably obtained by a standard assay (e.g., wet chemistry method).
- Feed nutrient concentrations may also be indirectly obtained. Because blood nutrient concentrations correlate with the ingested amount of the nutrient, blood nutrient concentrations (e.g., measured values) may be used to deduce a feed nutrient concentration. If a concentration is deduced in such manner from measured value(s), the expression "deduced concentration" may be used. In an exemplary parameterized model as described in this specification, the deduced feed nutrient concentration is a dependent variable in a regression analysis whose value can be deduced from independent variables such as blood nutrient concentration and values of parameters of the corresponding nutritional log. The term 'estimated' is used in this specification as a synonym for 'deduced' and vice versa.

- Biomarker concentrations may also be indirectly obtained. Because blood nutrient concentrations correlate with the ingested amount of the nutrient, nominal feed concentrations (e.g., expected values given a nutritional log) may be used to deduce a biomarker concentration. If a concentration is deduced in such manner from measured value(s), the expression "deduced concentration" may be used. In another exemplary model as described in this specification, the deduced biomarker concentration is a dependent variable in a regression analysis whose value can be deduced from independent variables such as feed nutrient concentration and values of parameters of the corresponding nutritional log. The term 'estimated' is used in this specification as a synonym for 'deduced' and vice versa.

- A model may be used to deduce e.g., feed nutrient concentrations from blood nutrient concentrations or vice versa. In the context of the present disclosure, a "model" is a mathematical model, which is preferably deterministic (e.g., will always produce the same output from a given starting condition or initial state).

- A "nutritional log" comprises information about how an animal has been fed so far. Preferably, it comprises the following information:

  • number of days the animal has been fed so far
  • amount of feed that is accessible to the animal per day (e.g., indicated in kg per day or ad libitum feeding)
  • the nominal or actual concentration of at least one nutrient that is comprised in the animal's feed (e.g., indicated as mg per kg feed)

- Unless indicated differently, a nutritional log may comprise data relating to multiple animals, provided all animals are of the same species and have been fed with the same feed in the same amount for the same number of days. Usually, this applies to animals of the same flock.

- Ideally, the actual concentration matches the corresponding nominal concentration and/or the corresponding deduced concentration. In this context, "corresponding" means that the concentrations being compared is interrelated (e.g., relate to the same nutrient/biomarker, relate to the same batch of feed, and relate to the blood of animals that have been fed with the same feed). The word "corresponding" flags situations where any unreasonable comparison of apples and oranges is excluded.

- Any meaningful discrepancy between the actual concentration and an estimated, calculated, or deduced concentration is a hint that something is not as it should be. In the context of the present disclosure, "discrepancy feedback" is information about any difference between the actual concentration of a (micro-)nutrient and an estimated, calculated or predicted concentration of the same (micro-)nutrient, either in the blood or in the feed of animal(s). Preferably, it is information about any difference between the nominal feed nutrient concentration and deduced feed nutrient concentration in regard of the same (micro-)nutrient. A discrepancy feedback may be negative in the sense that there is no discrepancy. Discrepancy feedback is preferably part of the herein disclosed feed surveillance.

- "Feed surveillance" is about monitoring the concentration of (micro-)nutrients in feed. In one embodiment of the present disclosure, feed surveillance is a process comprising the steps (1) receiving data, (2) deducing the feed nutrient concentration from the received data, (3) comparing the deduced feed nutrient concentration with the nominal feed nutrient concentration, (4) providing discrepancy feedback, and optionally (5) providing a recommendation on how to resolve any observed discrepancy. An exemplary recommendation could be to increase the concentration of a specific (micro-)nutrient in the animal's feed or drinking water. Another exemplary recommendation could be to improve the mixing process such that a feed additive is more homogenously distributed within the prepared feed. A further exemplary recommendation could be to treat animals such that bioavailability of the ingested (micro-)nutrients is increased (e.g., treating the animal's diarrhea). When implementing the method relating to feed surveillance and raising animals, recommendations are most often based on the response of a blood biomarker to feed that has been ingested by the animal whose blood has been analyzed. Therefore, recommendations are preferably referred to as "response based nutrient recommendation". The expression "response based nutrient recommendation" is to be understood as "response based (micro-)nutrient recommendation" (e.g., the recommendation relates preferably to a micronutrient). In the context of the present disclosure, recommendations are preferably the output of a software application.

- In the context of birds, the abbreviation "d1" refers to day 1 post-hatch. This applies mutatis mutandis to similar abbreviations such as "d2 (referring to day 2 post-hatch), "d3" (referring to day 2 post-hatch) etc. Consequently, the notation "d1-15" refers to a time span from 1 day to 15 days of age. Notations such as "d1-15" and "d1-d15" are used interchangeably.

- A "diet" refers to feed that is adapted to the needs of a specified population of animals. By way of example, feed that has been adapted to the needs of broilers from 1 day to 14 days of age is sometimes referred to as starter diet.

- A "set-up" is a way in which things are arranged. In a preferred embodiment of the disclosure, the set-up comprises a computer system, at least one point-of-care device at least one water flow line and at least one apparatus for metering nutrients into a water flow line. In a preferred embodiment of the disclosure, some or all of these three elements are operatively linked. By way of example, the point-of-care device may send data to the computer system and/or may receive data from the computer system, whereas the computer system may send data to the apparatus for metering. These three elements may be located on a farm where animals are raised. The set-up of the disclosure may comprise further elements, e.g. a stock of nutrients.

- The apparatus for metering nutrients is preferably a medicator. A "medicator" is an apparatus (preferably an injector) for introducing a drug or other substance into a liquid supply line in controlled amounts. Preferred medicators withdraw a set amount of a solution (e.g. water comprising electrolytes) and injects it into the water lines of the farm. The target animals then consume the solution over time. Thereby, the solution to water ratio can be adjusted. A typically ratio may be 1:128, i.e. 1 oz. of solution to 128 oz. (1 gal.) of water.

### 1. Model-Based Detection of Deficiency in Animal's Nutritional State

**Fig. 1** schematically shows a computer system 100 for detecting a deficiency in a nutritional state of at least one animal. The computer system 100 may comprise an input interface subsystem 120 which may be configured to access various types of data as described in this specification. For example, the input interface subsystem 120 may access blood measurement data 22 generated by a point-of-care device 20, temperature data 42 generated by a temperature sensor 40, and/or a data representation of a parameterized model 142. For that purpose, the input interface subsystem 120 may comprise one or a number of physical and/or virtual interfaces, with the number and respective types of interfaces depending on a source of the data. For example, for data accessible via network, the input interface subsystem 120 may comprise or may be constituted by network interface, such as a wired communication interface (e.g., Ethernet, fiber-optic based) or a wireless communication interface (e.g., Wi-Fi) or a virtual, software-based network interface. For data stored on an internal data storage, the input interface subsystem 120 may comprise or may be constituted by a data storage interface, such as a memory or hard drive or solid-state disk interface. For data accessed directly from a device or a sensor, such as the aforementioned point-of-care device 20 or the temperature sensor 40, the input interface subsystem 120 may comprise or may be constituted by a physical or virtual interface which allows communication with the device and/or the sensor. For temporary or longer-term storage of any data, the computer system 100 may comprise a data storage 140, which may for example comprise a persistent memory, a hard drive, a solid-state disk, etc.

The computer system 100 may further comprise a processor subsystem 160 which may be configured to perform the functions as described in this specification, including but not limited to determining, from the blood measurement data, a measured concentration of at least one biomarker associated with a nutritional state of the at least one animal, and detect a deficiency in the nutritional state of the at least one animal using a parameterized model, the measured biomarker concentration and a nominal concentration of the feed nutrient in the feed of the at least one animal. The processor subsystem 160 may generate output data, which output data may include display data 192 which may be output on a display 80 via a display interface 190, and actuator control data 182 which may be provided to an actuator 60 via an actuator interface 180. The actuator may for example be an actuator of a dispensing system (not shown in Fig. 1) which is configured to dispense nutritional supplements to the animals. It will be appreciated that various other types of outputs and output interfaces are equally conceivable. For example, instead of a display, another type of sensory perceptible output device may be used.

The computer system 100 may be part of a larger system for detecting a deficiency in a nutritional state of animals. In some examples, this larger system may comprise several cooperating parts, such as the computer system 100 and the point-of-care device 20 and the actuator 60 (or the dispensing system with the actuator).

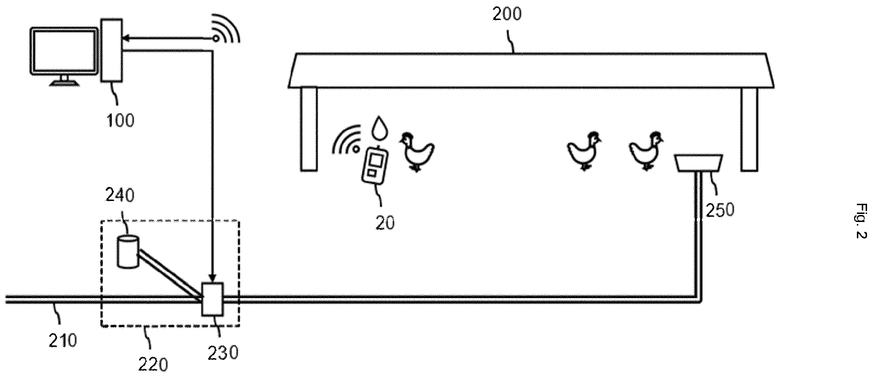

**Fig. 2** shows the computer system 100 on premise in a farm, which farm is schematically represented in Fig. 2 by a building 200 for housing animals, with the animals being in this specific example broiler housed in a broiler house. The point-of-care device 20 may be used to perform ex vivo blood measurements on select animals and transmit the blood measurements wirelessly to the computer system 100. The computer system 100 may be configured to identify, from the blood measurement data, a concentration of at least one biomarker associated with a nutritional state of the animal(s), and estimate the concentration of the feed nutrient in the feed of the animal(s) by using the concentration of the biomarker as input to a parameterized model, or estimate the biomarker concentration using the nominal concentration of the feed nutrient in the feed of the animal(s) as input to the parameterized model. If a deficiency is detected, e.g., compared to a reference value in the respective domain (being the measured biomarker concentration in the biomarker domain and the nominal feed concentration in the feed domain), the computer system 100 may automatically or semi-automatically (e.g., conditional to approval by a user) dispense nutritional supplements. This may involve controlling an actuator of a dispensing system 220. In the example of Fig. 2, the dispensing system 220 is shown to comprise an actuator in the form of a metering device 230 which may dispense nutritional supplements from a nutrient reservoir 240 into a water line 210, which water line 210 may then continue to a drinking water dispenser 250 (shown schematically in Fig. 2). For example, the nutrient reservoir 240 may contain nutrients which may be directly dispensed by the metering device 230 into the drinking water. In other examples, the nutrient reservoir 240 may contain an aqueous solution of nutritional supplements, which aqueous solution may then be dispensed into the drinking water by the metering device 230. In some examples, the computer system 100 may additionally or alternatively control the preparation of the aqueous solution, e.g., by controlling the concentration of nutrients therein.

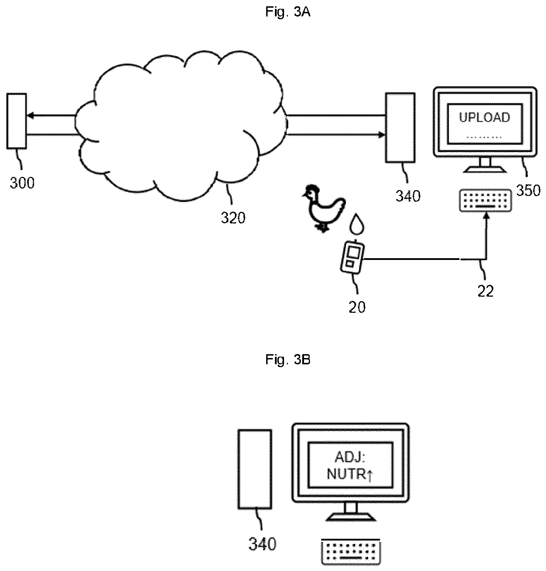

**Figs. 3A** and **3B** show a client/server implementation of the computer system. Here, a server 300 is provided which may be located remotely from the farm, while a client device 340 is provided on premise or nearby the farm, or in general, nearby the location of the animals. The client device 340 may for example be a workstation, a laptop, a tablet device, or a smartphone, and may be configured to communicate with the server 300 via a network 320. In particular, the client device 340 may be configured to act as an input device and output device for the server 300, allowing a user to provide input data and control aspects of the calculations performed by the server 300 and to obtain feedback in the form of output data. In the specific example of Figs. 3A and 3B, the server 300 is shown to be network-accessible via a network 320, such as the Internet, and the client device 340 is shown to comprise or be connected to a display 350 and configured to establish a graphical user interface on the display 350 which allows a user, such as the farmer, to upload blood measurement data 22 obtained from a point-of-care device 20. This may for example comprise the user manually entering the blood measurement data, or the user effecting an automatic transfer of the blood measurement data 22 to the server 300, e.g., by selecting the point-of-care device as a data source and by requesting a start of the upload. In this respect, it is noted that not all of the blood measurement data acquired by the point-of-care device 20 needs to be uploaded. For example, if it is known that only select biomarker(s) are of interest, the user may only manually enter concentrations of these biomarker(s), or the client device 340 may only select those concentrations for upload. As is shown in Figs. 3A and 3B, the user may thus upload blood measurement data to the server 300 and may receive feedback from the server, which is shown in Fig. 3B to be a recommendation to increase the amount of nutrients provided to the animals.

In some examples, the server 300 may be configured to perform the computer-implemented steps as described in this specification, such as those of the computer system 100 of Figs. 1 and 2. In other examples, the computer-implemented steps as described in this specification may be distributed over the server 300 and the client device 340. In some examples, the server 300 may be configured to establish a web-accessible graphical user interface, e.g., via a webpage. In such examples, a client device may not need to install specific software to make use of the functionality of the server 300. In other examples, client software may be installed on the client device to access the functionality of the server 300 via the client software. For example, such client software may be an application, or in short, an 'app', for the client device. In such cases, the functionality of the computer system as described elsewhere may be embodied by the server or the server and client device together.

In general, each computer system described in this specification, including the server and client device, may be embodied as, or in, a single device or apparatus, such as a workstation or a server. The device or apparatus may comprise one or more microprocessors which execute appropriate software. For example, the processor subsystem of a respective computer system may be embodied by a single Central Processing Unit (CPU), but also by a combination or system of such CPUs and/or other types of processing units. The software may have been downloaded and/or stored in a corresponding memory, e.g., a volatile memory such as RAM or a non-volatile memory such as Flash. Alternatively, the processor subsystem of a respective computer system may be implemented in the device or apparatus in the form of programmable logic, e.g., as a Field-Programmable Gate Array (FPGA). In general, each functional unit of a respective computer system may be implemented in the form of a circuit. A respective computer system may also be implemented in a distributed manner, e.g., involving different devices or apparatuses, such as distributed local or cloud-based servers.

## 1) Feed surveillance

The following discusses the use of the computer system and computer-implemented method as described in this specification for feed surveillance. Nutrient requirements of animals are known. Feed is formulated to meet the nutrient requirements of the animal that will be fed. Most often, feed is a mixture of multiple feed ingredients. The recipe for such mixture is typically provided by a nutritionist who is familiar with nutrient requirements of animals. In feed mills, feed is then prepared by mixing the feed ingredients according to the nutritionist's recipe.

A particular nutrient (for example Na+) may be present in more than one feed ingredient. Thus, when calculating a nominal feed nutrient concentration, all feed ingredients containing the respective nutrient may need be considered. To do so, computer software is available (input: feed recipe; output: list of the most important nutrients, including nominal feed nutrient concentrations). Such computer software may be accessed as a database, or the relation between feed recipe and nutrients and their concentration may be directly stored in a database. Thanks to such a database, nominal concentrations of many nutrients are obtainable at the push of a button. Preferably, one of the following software is used: Brill Formulation (Feed Management Systems, Inc), Concept 5 (Creative Formulation Concepts), Excel (Microsoft), Spesfeed Express (Spesfeed Consulting (PTY) Ltd).

Formulating feed may be an iterative process: the nutritionist is modifying the recipe of the fed to be manufactured until the nominal feed nutrient concentrations (as provided by the software) meet the assumed requirements of the animals that will be fed. In that sense, nominal feed nutrient concentrations are target values which may or may not be met by the feed that will eventually be manufactured in the feed mill. The purpose of feed surveillance is to make sure that actual feed nutrient concentrations match the respective nominal feed nutrients concentrations.

Feed surveillance could be done by comparing actual feed nutrient concentrations with the respective nominal feed nutrient concentrations. To do so, actual feed nutrient concentrations would have to be measured systematically in specialized laboratories using standard assays. Such approach is complicated, slow, and costly. Unless blind flying seems acceptable, not doing any feed surveillance is not an option either. Thus, there is a need for a feed surveillance system whose attributes are simplicity, sensitivity, timeliness, and cost.

Surprisingly, a simple, sensitive, cost effective and fast feed surveillance is at hand when using system and computer-implemented method as described in this specification to deduce feed nutrient concentrations from blood measurement data of a point-of-contact device and using a parameterized model. In such embodiments, the parameterized model may be generated to using the concentrations of the feed nutrient in feed fed to animals as one or more independent variables and the concentrations of the biomarker in blood of at least a subset of the animals as one or more dependent variables, so that an input of a measured biomarker concentration yields an estimated or deduced feed nutrient concentration. If a deduced feed nutrient concentration matches the corresponding nominal feed nutrient concentration, there is no longer any need to measure the actual feed nutrient concentration. Thus, no cumbersome lab analysis of feed needs to be done. Cost is reduced and the information about quality aspects of the manufactured feed is obtained much quicker. Feed nutrient concentrations may be deduced from blood nutrient concentrations. Since blood nutrient concentrations can be measured on the spot (e.g., on the farm, within minutes) with commercially available point-of-care devices, such an approach is simple, fast, relatively cheap, and sensitive.

Many commercially available point-of-care devices are suitable to measure the concentration of a nutrient, of a metabolite of a nutrient or of any other biomarker by which a feed nutrient concentration can be deduced. Most often, measurements are done *ex vivo* in a sample of the animal's blood. Preferred point-of-care devices are:

- i-Stat® Alinity v handheld blood analyzer fitted with a Chem8+ cartridge (Abbott Point of Care Inc., Princeton, NJ)
- iCheck® carotene photometer device and test kit (BioAnalyt GmbH, Potsdam, Germany)
- Vetscan® VS2 Chemistry Analyzer (Abaxis, inc) using the Avian/Reptilina Profile Plus cartridge (Abbott Point of Care Inc., Princeton, NJ)

Some embodiments relate to the use of at least one point-of-care device for doing feed surveillance, wherein said point-of-care device is suitable for measuring the concentration of at least one biomarker in the blood of an animal. Preferably, said at least one point-of-care device is suitable for measuring *ex vivo* the concentration of at least one biomarker in the blood of a monogastric animal. Preferred biomarkers are nutrients, micronutrients, and metabolites thereof.

Deducing feed nutrient concentrations from blood nutrients concentration is surprising because it is based on retrodiction. Retrodiction is the act of making a prediction about the past. In the context of the present disclosure, nutrient concentrations in feed that has been fed in the past may be predicted by blood nutrient concentrations that are measured in the present. Thus, instead of measuring nutrient concentrations in the feed of the past, nutrient concentrations may be predicted by presently measured blood nutrient concentrations. The correctness of the prediction may be checked by at any time by drudgingly measuring the actual feed nutrient concentration in the feed of the past, for example using wet chemistry methods. Stocking a sample of any feed batch is therefore preferred.

The preferred nutrient which is estimated by the parameterized model is a micronutrient and/or is an ion, a vitamin, a carotenoid, the total protein content or myo-inositol. In case of an ion, said ion is preferably selected from the group consisting of sodium, potassium, chloride, phosphorus, and calcium, wherein said ion is preferably not a trace mineral. In case of a vitamin, said vitamin is preferably selected from the group consisting of vitamin A, vitamin C, vitamin D and vitamin E.

When doing feed surveillance as herein disclosed, feed nutrient concentrations may be deduced from blood nutrient concentrations. In many cases, blood nutrient concentrations correlate over a large range with the amount of (micro-)nutrient that has been ingested by the animal whose blood nutrient concentration is measured. Therefore, blood nutrient concentrations may be converted into corresponding feed nutrient concentrations, the latter being referred to as deduced feed nutrient concentrations. This conversion may be done by providing a parameterized model which may be generated by data fitting to the experimental data. Such data fitting may for example comprise regression analysis of the experimental data. If the regression analysis, or in general data fitting, is done using machine learning, such experimental data may also be referred to as 'training data'.

In its simplest version, the parameterized model may be a linear function following the general scheme of

$$feed\ nutrient\ concentration = f(blood\ nutrient\ concentration)$$

$$= a \cdot blood\ nutrient\ concentration + b$$

wherein a is the slope and b is the y-intercept. The parameterized model may represent the function f and may directly comprise as parameters the parameters a and b, or may comprise other parameters which indirectly represent the parameters a and b. Such parameters may be manually determined, but also automatically, e.g., by linear regression analysis. In some cases, the relationship might be more complex, e.g., when blood is a transient pool and the respective nutrients are stored in bone, muscle or support organs/viscera. In such cases, non-linear regression analysis may be used to model this relation by a non-linear function. The parameterized model may then represent this non-linear function, with the parameters having been determined by the non-linear regression analysis, e.g., using polynomial regression. The parameterized model may for example take the form of a neural network, a random forest, a support vector machine, etc., with the parameters of the model (e.g., weights of a neural network) directly or indirectly modelling the relation between independent and dependent variables. In some cases, a linear or non-linear function may also be approximated by parameterized model, e.g., by weights of a neural network being trained to approximate a non-linear function between the blood nutrient concentration and the feed nutrient concentration.

The link between nutrients/feed intake and blood may also be subject to nutrient and hormonal regulation. For example, blood glucose concentrations are controlled by insulin and glucagon. Many post-absorptive changes, hormonal and/or transient responses are known and can be taken into account by more complex mathematical functions and models. More complex mathematical functions are accessible for the person skilled in the art using Excel (Microsoft) or preferably a statistical software program such as JMP (SAS), SAS, R (R Foundation for Statistical Computing) and SPSS (SPSS, Inc), which may be used to generate the parameterized model. Alternatively, a machine learnable model, such as a neural network, a random forest, a support vector machine, may be trained using known training techniques, for example using a gradient descent optimization technique.

In this respect, it is noted that the parameterized model may in some embodiments receive a biomarker concentration as input and provide a feed nutrient concentration as output, or vice versa: receive a feed nutrient concentration as input and provide a biomarker concentration as output. In some examples, the parameterized model may allow the input and output to be reversed, e.g., to input a biomarker concentration to obtain a feed nutrient concentration as output and to input feed nutrient concentration to obtain a biomarker concentration as output. For example, the parameterized model may model a bijective function and may thereby be inversible. Determining an inverse of a bijective function is known in the art. Also a machine learned function may allow the input and output to be reversed.

In some embodiments, (micro-)nutrients whose blood concentrations are capped may be excluded. Thus, an alternative embodiment of the present disclosure relates to the use of at least one point-of-care device for doing feed surveillance, wherein said point-of-care device is suitable for measuring the concentration of at least one nutrient, micronutrient or metabolite thereof, said at least one nutrient, micronutrient or metabolite being preferably an ion, a vitamin, a carotenoid, the total protein content and/or myo-inositol, with the proviso that nutrients, micronutrients and metabolites are excluded whose blood concentrations are capped (for example due to post-absorptive changes, hormonal and/or transient responses).

If the use of the parameterized model reveals that there is a discrepancy between a deduced feed nutrient concentration and the corresponding nominal feed nutrient concentration, there is a good chance that the underlying reason relates to a feed ingredient, and/or to the manufacturing process of the feed. However, there are other possible reasons for the revealed discrepancy, in particular a lower-than-normal bioavailability (e.g., due to diarrhea). If bioavailability is low, then the blood nutrient concentration might also be low, despite a correct nutrient concentration in the feed. As a consequence, a too low feed nutrient concentration may be then deduced from the corresponding blood nutrient concentration. The output data provided by the system and computer-implemented method may therefore be a recommendation to find out the probable cause of the revealed discrepancy. Such a recommendation may be given on-screen, e.g., via a graphical user interface.

In the context of feed surveillance, issues relating to bioavailability are preferably to be ruled out. To do so, the actual nutrient feed concentration may be measured in a laboratory using, e.g., by wet chemistry method. If the actual feed nutrient feed concentration is higher than the deduced feed nutrient concentration, there is good probability that the reason for the revealed discrepancy is a too low bioavailability e.g., due to diarrhea. This is summarized in below table:

| | Col 1 Discrepancy between **deduced** feed nutrient concentration and **nominal** feed nutrient concentration (yes/no) | Col 2 Discrepancy between **nominal** feed nutrient concentration and **actual** feed nutrient concentration (yes/no) | Possible interpretation |
|---|---|---|---|
| Feed #1 | yes | yes | The discrepancy between nominal and actual concentration had been correctly |
| | | | foreseen (cf. Col 1) before the actual feed nutrient concentration has been determined by a specialized laboratory (cf. Col 2). Feed #1 is likely to have an issue (e.g., poor quality of a feed ingredient, poor mixing process etc.). |
| Feed #2 | yes | no | There is no discrepancy between nominal and actual concentration (cf. Col 2). Thus, on feed level, there is no issue. Thus, the discrepancy between the deduced and the nominal concentration (cf. Col 1) suggests that there might be a bioavailability issue. In case the animal suffers from diarrhea, the deduced concentration is lower than the nominal/actual concentration. |
| Feed #3 | no | no | Everything is as it should be within measurement limits, deduced concentration = nominal concentration = actual concentration<br>The confirmation that nominal concentration (e.g., a target value) has indeed been reached by the actual confirmation (cf. Col 2) is a nice-to-have only. Next time, measurement of the actual feed nutrient concentration by a specialized lab can be omitted; this will save time and cost. |

To rule out any bioavailability issue, a preferred recommendation is to have measured the actual feed nutrient concentration of the respective feed batch by a specialized laboratory. Thus, a recommendation may be provided to measure the actual feed nutrient concentration of the nutrient in the animal's feed, and wherein said measurement is preferably done in a laboratory using a wet chemistry method.

In general, blood nutrient concentrations of more than one animal may be received. If data regarding multiple animals is received, an average of the animals' blood nutrient concentrations may be calculated. The thus obtained average may be input to the parameterized model to deduce a more reliable feed nutrient concentration. It goes without saying that calculating an average is only meaningful if all the animals are of the same or similar species and have received the same or similar feed in the same or similar amount. This applies, e.g., to animals of the same flock.

In general, the nutritional log of at least one animal may be taken into account in deducing the feed nutrient. Said nutritional log may comprise data about the animal's feed, including the nominal feed nutrient concentration of the animal's feed. Blood nutrient concentrations of the animal may be part of the nutritional log or may be provide separately once a blood sample of the animal has been examined *ex vivo* with the herein described point-of-care device.

If the actual feed nutrient feed concentration (e.g., the measured value) does not match the deduced feed nutrient concentration, the cause for any discrepancy determined using parameterized model is most likely the feed (e.g., not a bioavailability issue). Accordingly, a recommendation on how to improve the hitherto feed or the hitherto feed manufacturing process may be provided. An exemplary recommendation on how to improve the hitherto feed is checking the quality of the feed ingredients that have so far been used; a poor quality of feed ingredients may result in an actual feed nutrient concentration that is lower than the nominal feed nutrient concentration. An exemplary recommendation on how to improve the hitherto feed manufacturing process is improving the mixing process in the feed mill such that feed additives are more homogeneously distributed within the feed.

It is noted that while the nutritional deficiency may be detected in the feed domain, e.g., by comparing the estimated feed nutrient concentration to the nominal feed nutrient concentration as described above, the nutritional deficiency may also be detected by estimating a biomarker concentration from the nominal feed nutrient concentration and comparing the measured and estimated biomarker concentrations to detect the nutritional deficiency. Other aspects as described above apply in such case.

## 2) Raising animals

Feed surveillance increases profitability of livestock or poultry production systems by optimization of the farm's nutritional management. Thus, when raising animals, feed surveillance is important. However, raising animals is broader than just feed surveillance. Apart from dealing with quality aspects of feed, raising animals is also about the animals as such. Clearly, this includes potential health issues such as diarrhea. In severe cases, diarrhea is relatively easy to discover. Often, a short walk through the stable will be sufficient to detect traitorously wet droppings. Mild diarrhea, on the other hand, is harder to detect. Animals suffering from mild diarrhea usually survive. Therefore, mild diarrhea is sometimes underestimated. The disease may nevertheless have a significant impact on the profitability of large livestock or poultry production systems, mostly due a lower-than-normal bioavailability of nutrients. Regardless how mild diarrhea is, ingested nutrients are no longer absorbed as they should be. Thus, even if concentrations of the respective nutrients in the feed are correct, affected animals might no longer get a sufficient amount of (micro-)nutrients.

There is a surprising link between feed surveillance and the discovery of bioavailability issues: measuring blood nutrient concentrations - fast, cost effective and reliable with the herein described point-of-care device. Measuring blood nutrient concentrations pays twice: 1) when using a retrodictive model to deduce nutrient concentrations in feed that has been fed to an animal, blood nutrient concentrations allow monitoring feed quality and 2) when comparing measured blood nutrient concentrations with predicted blood nutrient concentrations, potential bioavailability issues can be revealed. Some embodiments relate to the use of at least one point-of-care device for raising animals, wherein said point-of-care device is suitable for measuring the concentration of at least one biomarker in the blood of an animal, and wherein said at least one biomarker is preferably indicative for the amount of a nutrient or a micronutrient that the animal has ingested, and wherein said animal is the source of the blood sample that is measured *ex vivo* with said point-of-care device.

A preferred embodiment relates to the use of the point-of-care device for monitoring bioavailability of at least one animal being raised in a livestock or poultry production system. An even more preferred embodiment of the present disclosure relates to the use of at least one point-of-care device for revealing diarrhea occurring in a flock of domesticated animals, wherein said domesticated animals are preferably monogastric animals, are preferably birds or pigs, and most preferably a chicken, turkeys, ducks, or geese, and wherein said chicken are preferably broilers.

If the measured blood nutrient concentration matches the predicted blood nutrient concentration (e.g., if the measured blood nutrient concentration is in a range of the predicted nutrient concentration or vice versa), everything seems fine. Most likely, the actual feed nutrient concentration matches the nominal feed nutrient concentration and most likely, the animal's bioavailability is as it should be. Thus, there is no need to alter the current situation on farm. However, if the measured blood nutrient concentration does not match the predicted blood nutrient concentration (e.g., the measured blood nutrient concentration is out of range of the predicted nutrient concentration or vice versa), further action may be needed. In this case, a recommendation is provided to the farmer or any other user. Because the recommendation is based on the response of an animal's blood biomarker to the animal's feed, the recommendation is referred to as response-based nutrient recommendation. Such response-based nutrient recommendation may for example be the output of a software application. One example of a computer-implemented method may comprise: accessing blood measurement data as described elsewhere; determining, from the blood measurement data, a measured concentration of at least one biomarker of a feed nutrient in feed of the at least one animal; providing a parameterized model as described elsewhere; obtaining a nominal or actual concentration of the feed nutrient in the feed of the at least one animal; estimating a concentration of the biomarker by using the nominal or actual concentration of the feed nutrient as input to the parameterized model; determining a difference between the measured concentration of the biomarker and said estimated concentration of the biomarker; and generating output data indicative of the difference.

The above method may be used to determine if the blood nutrient concentration is in range or is out of range of what is expected given the feed fed to the animal. As output, a response-based nutrient recommendation may be given if the blood nutrient concentration is out of range. If the blood nutrient concentration is out of range, there is a certain chance that the underlying reason relates to a feed ingredient, and/or to the manufacturing process of the feed. The alternative plausible reason is a lower-than-normal bioavailability (e.g., due to diarrhea). If bioavailability is lower than normal, the blood nutrient concentration will out of range even if the fed feed had correct feed nutrient concentrations. The recommendation might therefore be to take measures find out why the blood nutrient concentration is out of range.

To distinguish between a potential feed issue and a potential bioavailability issue, the actual nutrient feed concentration may be measured in a laboratory using e.g., by wet chemistry method. If the actual feed nutrient feed matches the nominal feed nutrient concentration, there is good probability that low bioavailability (e.g., due to diarrhea) is the reason why the blood nutrient concentration is out of range.

### 3) Heat stress

In hot weather, animals often hyperventilate in order to regulate core body temperature. This can lead to respiratory alkalosis due to the excessive loss of CO2 via the lungs and a rise in blood pH. In response, kidneys regulate the excretion of carbonate and hydrogen to control blood pH within physiologically acceptable ranges. The indirect effect of this is that blood potassium and sodium concentrations decrease and chloride increases. In anticipation thereof, feed having a comparatively low dEB is preferably fed to broilers and other livestock species. For example, broiler may be given feed which has a dEB from 100 to 170 milliequivalents (mEq), preferably from 100 to 145 milliequivalents (mEq). Feed with a low dEB has a tendency to cause metabolic acidosis which will compensate for a potential respiratory alkalosis triggered by heat stress. In other words, acidosis (e.g., low pH) compensates for alkalosis (e.g., high pH) and vice versa. Feed with a low dEB comprises normal potassium and/or sodium concentrations, and importantly, relatively low chloride concentration. Whereas low chloride concentration mitigates the negative impact of potential respiratory alkalosis, reducing chloride concentration in feed is somewhat risky. Chloride deficiency results in stunted growth, nervous symptoms, and various metabolic disorders. Thus, if the occasional hot spell does not strike as expected, measures should be taken to compensate for the relatively low dEB of the feed.

According to the present disclosure, blood concentrations of sodium, potassium and/or chloride may be monitored by measuring blood samples ex vivo. To do so, a commercially available point-of-care device may be used. If the expected hot spell is absent, the amount of Cl- in the prepared low dEB might not be sufficient and therefore, an aqueous solution comprising Cl- may be added to the drinking water, for example using a medicator. If chloride blood concentrations increase due to heat stress, no action may be needed because the amount of Cl- in the prepared low dEB was sufficiently low to compensation for the chloride increase triggered by heat stress.

Heat stress may lower potassium and sodium blood concentrations. If potassium and/or sodium blood concentrations decrease due to heat stress, an aqueous solution comprising Na+ and/or K+ may be added into the drinking water, preferably using a medicator. If the expected hot spell is absent, the amount of Na+ and/or K+ in the prepared low dEB might be sufficient.

Thus, necessary adjustments may be made by supplementing the drinking water of broilers and other animals. As these adjustments are complex, reliable information on electrolyte intake and/or on animal response are collected by measuring blood concentrations. Measurements may be done where animals are being raised (e.g., on the farm, using a point-of-care device). This allows to control interventions to be made. In particular, a deficiency in K+, Na+ and/or Cl- in the blood of an animal may be detected using the system and method as described in this specification. Namely, the amount of Na+, K+ and/or Cl- to be metered into animal's drinking water may be determined by measuring ex vivo the animal's biomarker concentration of Na+, K+ and/or Cl-, using a suitable point-of-care device, e.g., in a manner as described elsewhere in this specification for a feed nutrient, with this feed nutrient being in this particular example Na+, K+ and/or Cl-. The measured blood concentrations may then be input to the parameterized model to determine the amount of Na+, K+ and/or Cl- to be metered into, for example, a water flow line. For metering, a medicator or other metering device may be used. A "medicator" is an apparatus (preferably an injector) for introducing a drug or another substance into a liquid supply line in controlled amounts. Medicators may withdraw a set amount of a stock solution (e.g., water comprising Na+, K+ and/or Cl-) and inject it into the water lines of the farm. The target animals may then consume the solution over time. Thereby, the solution to water ratio can be adjusted.

In general, there are various manners how an aqueous stock solution can be added to drinking water of animals. In case of birds (e.g., broilers), the aforementioned medicator is preferably used to inject an aqueous stock solution comprising Na+, K+ and/or Cl- into drinking water flowing in a water flow line that is used to water the flock of birds. Thereby, said aqueous stock solution is added to the drinking water at a predetermined ratio of the aqueous stock solution to drinking water. When using a commercially available medicator, the predetermined ratio of the aqueous stock solution to drinking water is preferably from 1:80 to 1:150 and is more preferably 1:100 or 1:128. The predetermined ratio of the aqueous stock solution to drinking water depends on various factors, including the concentration of the aqueous stock solution and the concentration of Na+, K+ and/or Cl- in the drinking water before supplementation. It is therefore preferred that the concentration of Na+, K+ and/or Cl- in the drinking water of the group of animals before supplementation is also received.

There may also be a dependence on the water to feed intake ratio of the birds in the shed. Birds drink usually approx. 2 liters of water per kg of feed consumed. However, the water to feed intake ratio of the birds can vary from around 1.8 to 3.5 or can even be around 1, depending on feed composition and ambient temperature. In a preferred embodiment of the disclosure, the expected water to feed intake ratio is taken into account, for example on the basis of the ambient temperature, which temperature may be measured using a temperature sensor or obtained from a weather forecast.

### 4) Experimental data

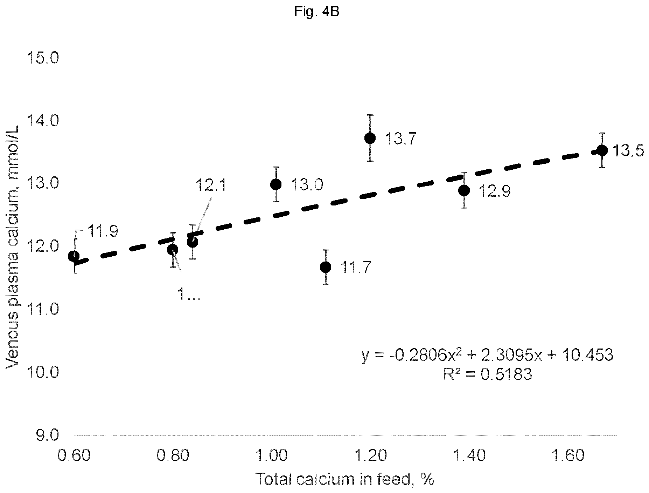

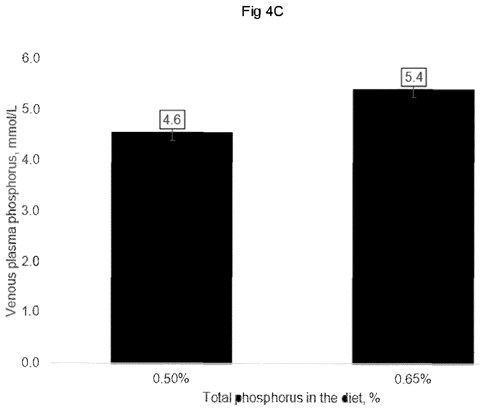

Figs. 4A-4D visualize data that was obtained in experimental example 1 (see below). **Fig. 4A** shows that ionized calcium (Ca2+) in the blood of broilers (y-axis: mmol/L) decreases as the total calcium in their diet (x-axis: weight-%, based on the total weight of the feed) decreases from 1.67 to 0.60 weight-%. Alternatively, calcium concentration could be expressed in g Ca2+ per kg feed (m/m). The relationship between the calcium contained in the prepared diets and the plasma calcium concentration can also be shown as depicted in Fig. 4B. In **Fig. 4B,** the amount of calcium in the diet is indicated on the x-axis, increasing from left to right. The y-axis shows total calcium in the blood (mmol/L). **Fig. 4C** shows that plasma phosphorus (inorganic phosphate as indicated by vetscan®; [PO4]3-) (y-axis: mmol/L) increases (P < 0.05) as total phosphorus in the diet increases from 0.50 to 0.65 weight-% (x-axis: weight-%, based on the total weight of the feed).

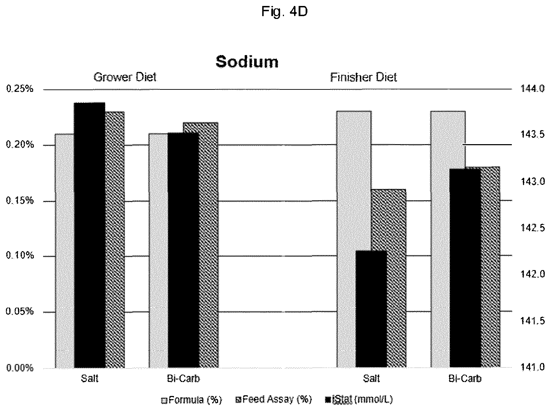

**Fig. 4D** relates to data that was obtained in example 2 and illustrates how formulated and analyzed dietary differences (e.g., discrepancies) can be detected by evaluating broiler blood physiology. In the left part of Fig. 4D, data relating to two variants of grower diet is shown. Data relating to two variants of finisher diet is shown on the righthand side. Na+ concentrations in feed are indicated in weight-%, based on the total weight of the feed (x-axis; left side). "Formula (%)" refers to a calculated, theoretical Na+ concentration in feed. "Feed Assay (%)" refers to a measured Na+ concentration in the feed. Values for Na+ concentration in blood are indicated in mmol per liter blood (x-axis; right side). "iStat (mmo/L)" refers to measured Na+ concentrations in blood, using a handheld iStat device. The right side of Fig. 4D shows that analyzed sodium values were lower than calculated/formulated. This discrepancy was detected by a statistical drop in broiler blood Na+ levels.

**Fig. 5** shows a flowchart of steps of a computer-implemented method 400 for detecting a deficiency in a nutritional state of at least one animal. The method may, but does not need to, correspond to an operation of the computer system 100 of Figs. 1-3B. All described variations of the computer system may be represented by corresponding variations of the method, and vice versa. The method comprises, in a step titled "ACCESS BLOOD MEASUREMENT DATA", accessing 410 blood measurement data as described elsewhere in this specification, and in a step titled "DETERMINE BIOMARKER CONCENTRATION", determining 420, from the blood measurement data, a measured concentration of a biomarker of a feed nutrient in feed of the at least one animal. The method further comprises, in a step titled "PROVIDE PARAMETERIZED MODEL", providing 430 a parameterized model as described elsewhere in this specification, in a step titled "OBTAIN NOMINAL CONCENTRATION OF FEED NUTRIENT", obtaining 440 a nominal concentration of the feed nutrient in the feed of the at least one animal, in a step titled "DETECT NUTRITIONAL DEFICIENCY USING PARAMETERIZED MODEL", detecting 450 a deficiency in the nutritional state of the at least one animal as described elsewhere in this specification, and in a step titled "GENERATE OUTPUT DATA", generating 460 output data as a function of the difference to enable the deficiency in the nutritional state to be addressed on the basis of the output data. In a specific example, as also shown in Fig. 5, the output data may be generated conditionally when the difference exceeds a threshold, e.g., if D(ifference) is larger than Th(reshold).

It will be appreciated that the above operations may be performed in any suitable order, e.g., consecutively, simultaneously, or a combination thereof, subject to, where applicable, a particular order being necessitated, e.g., by input/output relations. Parts of the method may be performed repeatedly. For example, as is also shown in Fig. 5, steps 410, 420, 440-460 may be repeated for each new blood measurement.

**Fig. 6** shows a flowchart of steps of a computer-implemented method 500 for generating a parameterized model for determining the nutritional state of animals. The method comprises, in a step titled "PROVIDE PARAMETERIZED MODEL", providing 510 a parameterized model as described elsewhere in this specification but having its parameters at a default or suboptimal value, in a step titled "PROVIDE BLOOD MEASUREMENT DATA", providing 520 blood measurement data representing one or more measured biomarker concentration in blood of one or more animals, in a step titled "PROVIDE NUTRIENT CONCENTRATION DATA", providing 530 one or more feed nutrient concentrations of feed fed to the animals within a same or related time period to which the blood measurement data pertains, in a step titled "MODEL DATA FITTING", data fitting 540 the parameterized model to experimental data comprising at least the feed nutrient concentrations and the measured biomarker concentration, and in a step titled "ADAPT PARAMETERS & OUTPUT MODEL", adapting 550 the parameters of the parameterized model in accordance with the data fitting and outputting a data representation of the parameterized model.

It will be appreciated that the above operations may be performed in any suitable order, e.g., consecutively, simultaneously, or a combination thereof, subject to, where applicable, a particular order being necessitated, e.g., by input/output relations. Parts of the method may be performed repeatedly. For example, different pairs of blood measurement data and nutrient concentration data may be accessed in steps 520, 530.

Each method, algorithm or pseudo-code described in this specification may be implemented on a computer as a computer implemented method, as dedicated hardware, or as a combination of both. As also illustrated in **Fig. 7,** instructions for the computer, e.g., executable code, may be stored on a computer-readable medium 600, e.g., in the form of a series 610 of machine-readable physical marks and/or as a series of elements having different electrical, e.g., magnetic, or optical properties or values. The executable code may be stored in a transitory or non-transitory manner. Examples of computer-readable mediums include memory devices, optical storage devices, integrated circuits, servers, online software, etc. Fig. 7 shows an optical disc 610.

In an alternative embodiment of the computer-readable medium 600, the computer-readable medium may comprise model data defining a parameterized model configured to model a relationship between concentrations of the biomarker and concentrations of the feed nutrient, as also described elsewhere in this specification.

## 2. *Use of Blood Biomarkers* to *Confirm Accuracy of Dietary Nutrient Supply to Production Animals*

The general remarks of or the embodiments in the previous sections and Figs. 1-7 may also apply here, but for conciseness they are not reiterated.

### 1) Feed surveillance

The present disclosure also relates to a computer-implemented method of doing feed surveillance, said method comprising the steps:

(i) receiving data,
(ii) using the data received in step (i) for providing the deduced feed nutrient concentration of a nutrient in animal's feed,
(iii) comparing the deduced feed nutrient concentration provided in step (ii) with the corresponding nominal feed nutrient concentration,
(iv) if a discrepancy has been revealed in step (iii), providing a recommendation on how to resolve the observed discrepancy, and

wherein the data received in step (i) comprises data sufficient to do steps (ii) and (iii), and
wherein said nutrient is preferably a micronutrient.

When doing feed surveillance as herein disclosed, the order of steps (i) and (ii) is irrelevant (e.g. step (ii) may be done before step (i), or step (i) and step (ii) may be done simultaneously). Step (iv) is preferred but optional.

The preferred nutrient of the present disclosure is a micronutrient and/or is a ion, a vitamin, a carotenoid, the total protein content or myo-inositol. In case of a ion, said ion is preferably selected from the group consisting of sodium, potassium, chloride, phosphorus and calcium, wherein said ion is preferably not a trace mineral. In case of a vitamin, said vitamin is preferably selected from the group consisting of vitamin A, vitamin C, vitamin D and vitamin E.

When doing feed surveillance as herein disclosed, feed nutrient concentrations are deduced from blood nutrient concentrations. In many cases, blood nutrient concentrations[1] see above definition of "blood nutrient concentrations". In case the nutrient is a micronutrient, "blood nutrient concentrations" is to be read as "blood micronutrient concentration". It is also noted, once again, that "blood nutrient concentration" is to be understood in a broad manner. In some cases, the concentration of a metabolite, of a ion and any other suitable blood biomarker is measured in blood. In blood, measuring the concentration of the (micro-)nutrient itself is not always meaningful or possible. correlate over a large range with the amount of (micro-)nutrient that has been ingested by the animal whose blood nutrient concentration is measured. Therefore, after suitable calibration, blood nutrient concentrations can be converted into corresponding [2] see above definition of "corresponding" The deduced feed nutrient concentration is the concentration of the (micro-)nutrient in the feed that has been fed to the animal whose blood was examined to obtain the blood nutrient concentration. feed nutrient concentrations [3] see above definition of "feed nutrient concentration". In case the nutrient is a micronutrient, "feed nutrient concentration" is to be read as "feed micronutrient concentration". , the latter being referred to as deduced feed nutrient concentrations. This conversion is done in step (ii) of the herein disclosed computer-implemented method of doing feed surveillance. In the easiest case, calibration shows that there is a linear relationship, following the general function $f(x)=a*x+b$. In some cases, the function might be more complex, e.g. when blood is a transient pool and the respective nutrients are stored in bone, muscle or support organs/viscera. The link between nutrients/feed intake and blood may also be subject to nutrient and hormonal regulation. For example, blood glucose concentrations are controlled by insulin and glucagon. Many post-absorptive changes, hormonal and/or transient responses are known and can be taken into account by more complex mathematical functions and models. More complex mathematical functions are accessible for the person skilled in the art using Excel (Microsoft) or preferably a statistical software program such as JMP (SAS), SAS, R (R Foundation for Statistical Computing) and SPSS (SPSS, Inc). Alternatively, (micro-)nutrients whose blood concentrations are capped may be excluded. Thus, an alternative embodiment of the present disclosure relates to the use of at least one point-of-care device for doing feed surveillance, wherein said point-of-care device is suitable for measuring the concentration of at least one nutrient, micronutrient or metabolite thereof, said at least one nutrient, micronutrient or metabolite being preferably an ion, a vitamin, a carotenoid, the total protein content and/or myo-inositol, with the proviso that nutrients, micronutrients and metabolites are excluded whose blood concentrations are capped (for example due to post-absorptive changes, hormonal and/or transient responses).

If step (iii) reveals that there is a discrepancy between a deduced feed nutrient concentration and the corresponding[4] see above definition of "corresponding" The deduced feed nutrient concentration and the corresponding nominal feed nutrient concentration relate to the same batch of feed. nominal feed nutrient concentration, there is a good chance that the underlying reason relates to a feed ingredient, and/or to the manufacturing process of the feed. However, there are another possible reasons for the revealed discrepancy - in particular a lower than normal bioavailability (e.g. due to diarrhea). If bioavailability is low, then the blood nutrient concentration might also be low, despite of a correct nutrient concentration in the feed. As a consequence, a too low feed nutrient concentration is then deduced from the corresponding blood nutrient concentration. The recommendation provided in step (iv) might therefore be to find out the probable cause of the revealed discrepancy.

To rule out any bioavailability issue, a preferred recommendation provided in step (iv) is to have measured the actual feed nutrient concentration of the respective feed batch by a specialized laboratory. Thus, a preferred embodiment of the disclosure relates to a computer-implemented method of doing feed surveillance, said method comprising the steps:

(i) receiving data, said data comprising the concentration of a nutrient or a metabolite thereof in the blood of an animal,
(ii) using the data received in step (i) for providing the deduced feed nutrient concentration of the nutrient in the animal's feed,
(iii) comparing the deduced feed nutrient concentration of step (ii) with the nominal feed nutrient concentration of the nutrient in the animal's feed, and
(iv) if a discrepancy has been revealed in step (iii), providing a recommendation,

wherein the recommendation of step (iv) is to measure the actual feed nutrient concentration of the nutrient in the animal's feed, and wherein said measurement is preferably done in a laboratory using preferably a wet chemistry method, and
wherein step (ii) is optionally done before step (i), or wherein step (i) and step (ii) are optionally done simultaneously, and wherein said nutrient is preferably a micronutrient, and wherein the data received in step (i) comprises any further data that is needed to do steps (ii) and (iii) such as the nominal feed nutrient concentration of the nutrient in the animal's feed.

"Comprising" is a term of art used in claim language which means that the named element is essential, but other elements may be added. Thus, in step (i), blood nutrient concentrations of more than one animal may be received. If data regarding multiple animals is received in step (i), an average of the animals' blood nutrient concentrations is preferably calculated. The thus obtained average can then be used in step (iii) to deduce a more reliable feed nutrient concentration. It goes without saying that calculating an average is only meaningful if all the animals are of the same species and have received the same feed in the same amount. This applies e.g. to animals of the same flock. Thus, a preferred embodiment of the disclosure relates to a computer-implemented method of doing feed surveillance, said method comprising the steps:

(i) receiving data, said data comprising the concentration of a nutrient or a metabolite thereof in the blood of an animal,
(ii) using the data received in step (i) for providing the deduced feed nutrient concentration of the nutrient in the animal's feed,
(iii) comparing the deduced feed nutrient concentration of step (ii) with the nominal feed nutrient concentration of the nutrient in the animal's feed, and
(iv) if a discrepancy has been revealed in step (iii), providing a recommendation,

wherein wherein blood nutrient concentrations from more than one animal of preferably the same flock are received in step (i) to calculate an average blood nutrient concentration, and wherein said average blood nutrient concentration is used in step (iii) to provide the deduced feed nutrient concentration, and
wherein step (ii) is optionally done before step (i), or wherein step (i) and step (ii) are optionally done simultaneously, and
wherein the recommendation of step (iv) is to measure the actual feed nutrient concentration of the nutrient in the animal's feed, and wherein said measurement is preferably done in a laboratory using preferably a wet chemistry method, and
wherein said nutrient is preferably a micronutrient, and
wherein the data received in step (i) comprises any further data that is needed to do steps (ii) and (iii) such as the nominal feed nutrient concentration of the nutrient in the animal's feed.

Preferably, the data received in step (i) comprises the nutritional log of at least one animal. Said nutritional log comprises data about the animal's feed, including the nominal feed nutrient concentration of the animal's feed. Blood nutrient concentrations of the animal may be part of the nutritional log or may be provide separately once a blood sample of the animal has been examined *ex vivo* with the herein described point-of-care device. Thus, an also preferred embodiment of the disclosure relates to a computer-implemented method of doing feed surveillance, said method comprising the steps:

(i) receiving data, said data comprising an animal's blood nutrient concentration that has been measured *ex vivo*, and said data further comprising the nutritional log of the animal whose blood nutrient concentration has been measured *ex vivo*, and

wherein said nutritional log comprises the nominal feed nutrient concentration of the nutrient in the animal's feed,

(ii) using the data received in step (i) for providing the deduced feed nutrient concentration of the nutrient in the animal's feed,

(iii) comparing the deduced feed nutrient concentration of step (ii) with the nominal feed nutrient concentration of step (i),

(iv) if a discrepancy has been revealed in step (iii), providing the recommendation to measure the actual feed nutrient concentration of the nutrient in the animal's feed, wherein said measurement is preferably to be done in a laboratory using preferably a by wet chemistry method, and optionally

(v) if there is no discrepancy between the measured actual feed nutrient feed concentration and the deduced feed nutrient concentration, providing a recommendation on how to improve the hitherto feed or the hitherto feed manufacturing process,

wherein optionally concentrations or data from more than one animal of preferably the same flock are received in step (i) to calculate an average, and wherein said average is then used in step (iii), and

wherein said nutrient is preferably a micronutrient, and

wherein the recommendation provided in step (iv) is to measure the actual feed nutrient concentration of the nutrient in the animal's feed, and wherein said measurement is preferably to be done in a laboratory using preferably a by wet chemistry method.

If the actual feed nutrient feed concentration (i.e. the measured value) does not match the deduced feed nutrient concentration, the cause for any discrepancy having been revealed in step (iii) is most likely the feed (i.e. not a bioavailability issue). Accordingly, a recommendation on how to improve the hitherto feed or the hitherto feed manufacturing process is then provided in optional step (v). An exemplary recommendation on how to improve the hitherto feed is checking the quality of the feed ingredients that have so far been used; a poor quality of feed ingredients may result in an actual feed nutrient concentration that is lower than the nominal feed nutrient concentration. An exemplary recommendation on how to improve the hitherto feed manufacturing process is Improving the mixing process in the feed mill such that feed additives are more homogeneously distributed within the feed.

In the context of the present disclosure, the preferred animal is a monogastric animal, whereas the preferred nutrient is a micronutrient. Thus, a preferred a computer-implemented method of doing feed surveillance comprises the steps:

(i) receiving data, said data comprising an animal's blood nutrient concentration that has been measured *ex vivo*, and said data further comprising the nutritional log of the animal whose blood nutrient concentration has been measured *ex vivo*, and

wherein said nutritional log comprises the nominal feed nutrient concentration of the nutrient in the animal's feed,

(ii) using the data received in step (i) for providing the deduced feed nutrient concentration of the nutrient in the animal's feed,

(iii) comparing the deduced feed nutrient concentration of step (ii) with the nominal feed nutrient concentration of step (i), and

(iv) if a discrepancy has been revealed in step (iii), providing a recommendation on how to resolve the observed discrepancy,

wherein said blood nutrient concentration is the concentration of a micronutrient or a metabolite thereof in the blood of a monogastric animal, and

wherein said monogastric animal is preferably a bird or pig, and is most preferably a chicken, a turkey, a duck or a goose, and wherein said chicken is preferably a broiler, and/or

wherein said micronutrient is preferably a ion or a vitamin, wherein said ion is preferably selected from the group consisting of sodium, potassium, chloride, phosphorus and calcium, and wherein said vitamin is preferably selected from the group consisting of vitamin A, vitamin C, vitamin D and vitamin E, and wherein said ion is preferably not a trace mineral, and wherein micronutrients whose blood concentrations are capped due to post-absorptive changes, hormonal and/or transient responses are excluded.

## 2) Computer-implemented method for deducing a feed nutrient concentration

In the context of the present disclosure, deduced feed nutrient concentrations play a relevant role. If a deduced feed nutrient concentration matches the corresponding nominal feed nutrient concentration, cumbersome determination of the actual feed nutrient concentration can be skipped.

Deducing feed nutrient concentrations from blood nutrients concentration is surprising because it is based on retrodiction. Retrodiction is the act of making a *prediction* about the past. In the context of the present disclosure, nutrient concentrations in feed that has been fed in the past are predicted by blood nutrient concentrations that are measured in the present. Thus, instead of measuring nutrient concentrations in the feed of the past, nutrient concentrations are predicted by presently measured blood nutrient concentrations. The correctness of the prediction could be checked by at any time by drudgingly measuring the actual feed nutrient concentration in the feed of the past. Stocking a sample of any feed batch is therefore preferred.

One embodiment of the present disclosure relates to a computer-implemented method for deducing a feed nutrient concentration, said method comprising the steps:

(i) receiving a blood nutrient concentration of an animal, wherein said blood nutrient concentration has been measured *ex vivo,* and receiving the nutritional log of the animal whose blood nutrient concentration has been measured *ex vivo,*
(ii) providing a model which allows to deduce a feed nutrient concentration from a blood nutrient concentration and a nutritional log, wherein said blood nutrient concentration has been measured *ex vivo* in the blood of an animal that has been fed according to the nutritional log with feed comprising the nutrient,
(iii) using the model of step (ii) to deduce the concentration of the nutrient in the feed the animal of step (i) has been fed with.

The general remarks of the previous section also relate to the herein disclosed computer-implemented method for deducing a feed nutrient concentration. Thus, step (ii) may be done before step (i), or step (i) and step (ii) may be done simultaneously. Similarly, blood nutrient concentrations of multiple animals may be received in step (i), provided all the animals have been fed with the same feed for the same amount of time and provided all the animals are of the same species. If these conditions are met, an average of the received blood concentrations nutrient concentrations can be used in step (ii).

The model of step (ii) is a mathematical model, i.e. uses mathematical concepts and language. Preferably, step (ii) is done on a computer system (e.g. on a desktop, laptop, smart phone or any other hand-held device) by running a software application. The software application may be installed on a computer system. Alternatively, the computer system may be used to access the software application through the internet and alike.

The purpose of the model of step (ii) is to deduce a feed nutrient concentration from a given blood nutrient concentration. Thus, the model of step (ii) is suitable for making a retrodiction or any other kind of prediction that involves moving backwards in time. From a given blood nutrient concentration and having the right model at hand, it can be deduced what the feed nutrient concentration must have been. In its simplest version, the model of step (ii) may be a linear function following the general scheme of

$$feed\ nutrient\ concentration = f(blood\ nutrient\ concentration)$$
$$= a \cdot blood\ nutrient\ concentration + b$$

wherein a is the slope and b is the y-intercept. The person skilled in the art knows how to find a values for a and b by doing suitable calibration experiments. Blood nutrient concentration correlates over a wide range with the nutrient concentration in the feed and thus, a model can always be provided by the person skilled in the art. If sufficient data is available, more sophisticated retrodictive models including a machine learning algorithm can be used in step (ii).

One embodiment of the present disclosure relates a computer-implemented method of doing feed surveillance, wherein a retrodictive model is used to deduce a nutrient concentration in feed that has been fed to an animal, and wherein said feed nutrient concentration is preferably deduced from the concentration of the same nutrient in the blood of said animal that has been fed with said feed.

The general remarks of the previous section apply. Thus, the preferred nutrient of the present disclosure is a micronutrient and/or is an ion, a vitamin, a carotenoid, the total protein content or myo-inositol. In case of an ion, said ion is preferably selected from the group consisting of sodium, potassium, chloride, phosphorus and calcium, wherein said ion is preferably not a trace mineral. In case of a vitamin, said vitamin is preferably selected from the group consisting of vitamin A, vitamin C, vitamin D and vitamin E. Again, the general remarks of the previous section apply and thus, nutrients and micronutrients whose blood concentrations are capped due to post-absorptive changes, hormonal and/or transient responses are preferably excluded. This applies to amino acids, carbohydrates, starches, fats and lipids. Alternatively, post-absorptive changes, hormonal and/or transient responses be part of a more sophisticated model provide in step (ii).

Regarding the preferred animals, the general remarks of the previous section also apply. Thus, the preferred animal is a monogastric animal, is preferably a bird or pig, and is most preferably a chicken, a turkey, a duck or a goose, and wherein said chicken is preferably a broiler animal.

Thus, a preferred embodiment of the present disclosure relates to a computer-implemented method for deducing a feed nutrient concentration, said method comprising the steps:

(i) receiving a blood nutrient concentration of an animal, wherein said blood nutrient concentration has been measured *ex vivo,* and receiving the nutritional log of the animal whose blood nutrient concentration has been measured *ex vivo,*
(ii) providing a model which allows to deduce a feed nutrient concentration from a blood nutrient concentration and a nutritional log, wherein said blood nutrient concentration has been measured *ex vivo* in the blood of an animal that has been fed according to the nutritional log with feed comprising the nutrient,
(iii) using the model of step (ii) to deduce the concentration of the nutrient in the feed the animal of step (i) has been fed with, and

wherein said blood nutrient concentration of an animal is the concentration of a micronutrient or a metabolite thereof in the blood of a monogastric animal, said animal being preferably a bird or pig, and wherein said micronutrient is preferably a ion or a vitamin.

### 3) Computer-implemented method of raising animals

Feed surveillance increases profitability of livestock production systems by optimization of the farm's nutritional management. Thus, when raising animals, feed surveillance is important. However, raising animals is broader than just feed surveillance. Apart from dealing with quality aspects of feed, raising animals is also about the animals as such. Clearly, this includes potential health issues such as diarrhea. In severe cases, diarrhea is relatively easy to discover. Often, a short walk through the stable will be sufficient to detect traitorously wet droppings. Mild diarrhea, on the other hand, is harder to detect. Animals suffering from mild diarrhea usually survive. Therefore, mild diarrhea is sometimes underestimated. The disease may nevertheless have a significant impact on the profitability of large livestock production systems, mostly due a lower than normal bioavailability of nutrients. Regardless how mild diarrhea is, ingested nutrients are no longer absorbed as they should be. Thus, even if concentrations of the respective nutrients in the feed are correct, affected animals might no longer get a sufficient amount (micro-)nutrients.

There is a surprising link between feed surveillance and the discovery of bioavailability issues: measuring blood nutrient concentrations - fast, cost effective and reliable with the herein described point-of-care device.

Measuring blood nutrient concentrations pays twice:

1. When using a retrodictive model to deduce nutrient concentrations in feed that has been fed to an animal, blood nutrient concentrations allow monitoring feed quality (cf. previous sections and Figure 8).
2. When comparing measured blood nutrient concentrations with predicted blood nutrient concentrations, potential bioavailability issues can revealed (Figure 9).

One embodiment of the present disclosure relates to the use of at least one point-of-care device for raising animals, wherein said point-of-care device is suitable for measuring the concentration of at least one biomarker in the blood of an animal, and wherein said at least one biomarker is preferably indicative for the amount of a nutrient or a micronutrient that the animal has ingested, and wherein said animal is the source of the blood sample that is measured *ex vivo* with said point-of-care device. A preferred embodiment of the present disclosure relates to the use of at least one point-of-care device for monitoring bioavailability of at least one animal being raised in a livestock production system, wherein said point-of-care device is preferably suitable for measuring the concentration of at least one biomarker in the blood of an animal, and wherein said at least one biomarker is indicative for the amount of a nutrient or a micronutrient that the animal has ingested, and wherein said animal is the source of the blood sample that is measured *ex vivo* with said point-of-care device. An even more preferred embodiment of the present disclosure relates to the use of at least one point-of-care device for revealing diarrhea occurring in a flock of domesticated animals, wherein said point-of-care device is preferably suitable for measuring the concentration of at least one biomarker in the blood of an animal, and wherein said domesticated animals are preferably monogastric animals, are preferably birds or pigs, and most preferably a chicken, turkeys, ducks or geese, and wherein said chicken are preferably broilers.

Over a relatively large range, the amount of ingested nutrient correlates with the observed blood nutrient concentration: the more of nutrient an animal ingests, the higher will be the concentration of the nutrient (or of a metabolite thereof) in the animal's blood. Provided sufficient data is available for calibration, the person skilled in the art can find values for parameters needed for a suitable mathematical prediction model. If the nutritional log of the animal is available, it is particularly straightforward to predict the concentration of a nutrient in the blood of an animal.

If the measured blood nutrient concentration matches the predicted blood nutrient concentration (i.e. if the measured blood nutrient concentration is in range), everything seems fine. Most likely, the actual feed nutrient concentration matches the nominal feed nutrient concentration and most likely, the animal's bioavailability is as it should be. Thus, there is no need to alter the current situation on farm.

However, if the measured blood nutrient concentration does not match the predicted blood nutrient concentration (i.e. the measured blood nutrient concentration is out of range), further action is needed. In this case, a recommendation is provided to the farmer or any other user. Because the recommendation is based on the response of an animal's blood biomarker to the animal's feed, the recommendation is referred to as response-based nutrient recommendation. Such response-based nutrient recommendation is preferably the output of a software application. The software application may be installed on a computer system. Alternatively, the computer system may be used to access the software application through the internet and alike. Any kind of computer system could be used, including smart phones and other handheld devices.

One embodiment of the present disclosure relates to a computer-implemented method of raising animals, said method comprising the steps:

(i) receiving a blood nutrient concentration of an animal, wherein said blood nutrient concentration has been measured *ex vivo,* and receiving the nutritional log of the animal whose blood has been examined *ex vivo,*

(ii) providing a model which defines blood nutrient concentrations that are in range if the animal whose blood is examined *ex vivo* has been fed for a predetermined period of time with a predetermined amount of feed, said feed comprising the corresponding nutrient in a given concentration,

(iii) using the model of step (ii) to determine if the blood nutrient concentration received in step (i) is in range or is out of range, and,

(iv) providing at least one response-based nutrient recommendation if step (iii) reveals that the blood nutrient concentration received in step (i) is out of range.

The general remarks of the previous section also relate to the herein disclosed computer-implemented method of raising animals. Thus, step (ii) may be done before step (i), or step (i) and step (ii) may be done simultaneously. Similarly, blood nutrient concentrations from more than one animal of the same flock may be received in step (i) to calculate an average blood nutrient concentration, and wherein said average is then used in step (iii)

The general remarks of the previous section also apply to the nutrient. Thus, the preferred nutrient is a micronutrient and/or is a ion, a vitamin, a carotenoid, the total protein content or myo-inositol. In case of a ion, said ion is preferably selected from the group consisting of sodium, potassium, chloride, phosphorus and calcium, wherein said ion is preferably not a trace mineral. In case of a vitamin, said vitamin is preferably selected from the group consisting of vitamin A, vitamin C, vitamin D and vitamin E. Again, the general remarks of the previous section apply and thus, nutrients and micronutrients whose blood concentrations are capped due to post-absorptive changes, hormonal and/or transient responses are preferably excluded. This applies to amino acids, carbohydrates, starches, fats and lipids. Alternatively, post-absorptive changes, hormonal and/or transient responses be part of a more sophisticated model provide in step (ii).

Regarding the preferred animals, the general remarks of the previous section also apply. Thus, the preferred animal is a monogastric animal, is preferably a bird or pig, and is most preferably a chicken, a turkey, a duck or a goose, and wherein said chicken is preferably a broiler animal.

If step (iii) reveals that the blood nutrient concentration received in step (i) is out of range, there is a certain chance that the underlying reason relates to a feed ingredient, and/or to the manufacturing process of the feed. The alternative plausible reason is a lower than normal bioavailability (e.g. due to diarrhea). If bioavailability is lower than normal, the blood nutrient concentration will out of range even if the fed feed had correct feed nutrient concentrations. The recommendation provided in step (iv) might therefore be to take measures find out why the blood nutrient concentration received in step (i) is out of range.

To distinguish between a potential feed issue and a potential bioavailability issue, the actual nutrient feed concentration can be measured in a laboratory using e.g. by wet chemistry method. If the actual feed nutrient feed matches the nominal feed nutrient concentration, there is good probability that low bioavailability (e.g. due to diarrhea) is the reason why the blood nutrient concentration received in step (i) is out of range.

In order to compare the measured feed nutrient concentration (i.e. the actual feed nutrient feed concentration) with the nominal feed nutrient concentration (i.e. with the target concentration), the two concentrations are to be provided. Each of these two concentrations may be provide before or after any one of steps (i) to (iv) of the herein disclosed computer-implemented method of raising animals. Preferably, the actual feed nutrient concentration is provided before any one of steps (i) to (iv), said actual feed nutrient concentration being the actual concentration of the nutrient in the feed of the animal whose blood has been examined *ex vivo.* Also preferably, the nominal feed nutrient concentration part of the nutritional log provided in step (i).

A preferred embodiment of the present disclosure relates to a computer-implemented method of raising animals, said method comprising the steps:

(i) receiving a blood nutrient concentration of an animal, wherein said blood nutrient concentration has been measured *ex vivo,* and receiving the nutritional log of the animal whose blood has been examined *ex vivo,*

(ii) providing a model which defines blood nutrient concentrations that are in range if the animal whose blood is examined *ex vivo* has been fed for a predetermined period of time with a predetermined amount of feed, said feed comprising the corresponding nutrient in a given concentration,

(iii) using the model of step (ii) to determine if the blood nutrient concentration received in step (i) is in range or is out of range, and

(iv) providing at least one response-based nutrient recommendation if step (iii) reveals that the blood nutrient concentration received in step (i) is out of range,

wherein the actual feed nutrient concentration is provided before any one of steps (i) to (iv), said actual feed nutrient concentration being the actual concentration of the nutrient in the feed of the animal whose blood has been examined *ex vivo,* and/or wherein the nominal feed nutrient concentration is provided before or after any one of steps (i) to (iv), said nominal feed nutrient concentration being preferably part of the nutritional log provided in step (i), and

wherein at least one measure to increase the nutrient's bioavailability in the animal is recommended in step (iv) in case a comparison between the actual feed nutrient concentration and the nominal feed nutrient concentration reveals that there is no discrepancy between the actual feed nutrient concentration and the nominal feed nutrient concentration, and

wherein said nutrient is a micronutrient and/or wherein said nutrient is a ion, a vitamin, a carotenoid, the total protein content and/or myo-inositol, and wherein said ion is preferably selected from the group consisting of sodium, potassium, chloride, phosphorus and calcium, and wherein said ion is preferably not a trace mineral, and wherein said vitamin is preferably selected from the group consisting of vitamin A, vitamin C, vitamin D and vitamin E.

FIG. 8 illustrates a specific embodiment of the disclosure, wherein the nominal feed nutrient concentration is compared with the deduced feed nutrient concentration. A model is provided that defines a range for a feed nutrient concentration (dependent variable), deduced from blood nutrient concentration (measured *ex vivo*) and values of the nutritional log's parameters (independent variables). In case the comparison reveals that there is no meaningful discrepancy, livestock breeding or livestock farming can continued in the same manner as it has been done before; there is no need for a costly and time consuming standard assay in highly specialist laboratory. On the other hand, if the comparison reveals a meaningful discrepancy, the same steps as described re Figure 8 follow. A comparison of Figure 8 and Figure 9 shows that the two specific embodiments are based on the same inventive concept.

FIG. 9 illustrates another specific embodiment of the disclosure, wherein the *ex vivo* determined blood nutrient concentration is compared with a deduced blood concentration of the same (micro-)nutrient. A model is provided that defines a range for a blood nutrient concentration (dependent variable), deduced from the nominal (micro-)nutrient concentration in the feed that has been fed and other values of the nutritional log's parameters (independent variables). In case the comparison reveals a meaningful discrepancy, a sample of the feed is analyzed to reveal the actual feed nutrient concentration. For this purpose, some feed has been stocked (i.e. the whole batch of feed has been fed). A response-based nutrient recommendation can then be provided. If the actual feed nutrient concentration is the same as the nominal feed nutrient concentration, the cause for the revealed discrepancy might be a low bioavailability, e.g. due to mild diarrhea. Adequate measures can then be taken. If the actual feed nutrient concentration is different from the nominal feed nutrient concentration, the cause for the revealed discrepancy might be a poor mixing process, poor quality of a feed additive, an operational error and alike. Again, adequate measures can then be taken to resolve the issue.

### 3. Improving Accuracy of Electrolyte Supply to Production Animals

The general remarks of or the embodiments in the previous sections and Figs. 1-9 may also apply here, but for conciseness they are not reiterated.

The present embodiments relate to the acid-base balance of livestock species such as broilers laying hens, turkeys, ducks, swine, horses, sheep and cattle. Chloride is one of the elements which contributes to the acid-base balance, the others being sodium and potassium.

In hot weather, animals often hyperventilate in order to regulate core body temperature. This can lead to respiratory alkalosis due to the excessive loss of $CO_2$ via the lungs and a rise in blood pH. In response, kidneys regulate the excretion of carbonate and hydrogen to control blood pH within physiologically acceptable ranges. The indirect effect of this is that blood potassium and sodium concentrations decrease and chloride increases. In anticipation thereof, feed having a comparatively low dEB is preferably fed to broilers and other livestock species. Feed with a low dEB has a tendency to cause metabolic acidosis which will compensate for a potential respiratory alkalosis triggered by heat stress. In other words, acidosis (i.e. low pH) compensates for alkalosis (i.e. high pH) and vice versa.

Feed with a low dEB comprises normal potassium and/or sodium concentrations, and importantly, relatively low chloride concentration. Whereas low chloride concentration mitigates the negative impact of potential respiratory alkalosis, reducing chloride concentration in feed is somewhat risky. Chloride deficiency results in stunted growth, nervous symptoms and various metabolic disorders. Thus, if the occasional hot spell does not strike as expected, measures should be taken to compensate for the relatively low dEB of the feed.

According to the present disclosure, blood concentrations of sodium, potassium and/or chloride are monitored by measuring blood samples *ex vivo*. To do so, a commercially available point-of-care device may be used.

If the expected hot spell is absent, the amount of Cl- in the prepared low dEB might be not be sufficient and therefore, an aqueous solution comprising Cl- may be added to the drinking water, preferably using a medicator. If chloride blood concentrations increases due to heat stress, no action may be needed because the amount of Cl- in the prepared low dEB was sufficiently low to compensation for the chloride increase triggered by heat stress.

Heat stress may lower potassium and sodium blood concentrations. If potassium and/or sodium blood concentrations decrease due to heat stress, an aqueous solution comprising Na+ and/or K+ may be added into the drinking water, preferably using a medicator. If the expected hot spell is absent, the amount of Na+ and/or K+ in the prepared low dEB might be sufficient.

Thus, necessary adjustments are made by supplementing the drinking water of broilers and other animals. As these adjustments are complex, reliable information on electrolyte intake and/or on animal response are collected by measuring blood concentrations. Measurements are done where animals are being raised (i.e. on the farm, using a point-of-care device). This allows to control interventions to be made.

The apparatus for metering nutrients is preferably a medicator. A "medicator" is an apparatus (preferably an injector) for introducing a drug or another substance into a liquid supply line in controlled amounts. Preferred medicators withdraw a set amount of a stock solution (e.g. water comprising Na+, K+ and/or Cl-) and injects it into the water lines of the farm. The target animals then consume the solution over time. Thereby, the solution to water ratio can be adjusted. A typical ratio may be 1:128, i.e. 1 oz. of solution to 128 oz. (1 gal.) of water.

In the context of the present disclosure, a group of animals are animals of a given species that receive the same feed, the same drinking water and are exposed to the same ambient air temperature. A "flock" is a group of animals comprising preferably at least 10, more preferably to at least 100 and most preferably to at least 1000 animals that are raised in the same compartment (e.g. in the same pen or in the same house). In a flock of animals, the animals have approximatively the same age. In case of birds, "approximatively the same age" means that age may vary by 1-2 days because batches of eggs put in the incubator will hatch from around 20-22 days after placement.

## 1) Computer-implemented method of supplementing the drinking water of a group of animals with at least one electrolyte

Over a relatively large range, the amount of ingested nutrient correlates with the observed blood nutrient concentration: the more of a nutrient an animal ingests, the higher will be the concentration of the nutrient (or of a metabolite thereof) in the animal's blood. Provided sufficient data is available for calibration, the person skilled in the art can find values for parameters needed for a suitable mathematical prediction model. If the nutritional log of the animal is available, it is particularly straightforward to predict the concentration of a nutrient in the blood of an animal.

If the measured blood nutrient concentration matches the predicted blood nutrient concentration (i.e. if the measured blood nutrient concentration is in range), everything seems fine. Most likely, the actual feed nutrient concentration matches the nominal feed nutrient concentration and most likely, the animal's bioavailability is as it should be. Thus, there is no need to alter the current situation on farm.

However, if the measured blood nutrient concentration does not match the predicted blood nutrient concentration (i.e. the measured blood nutrient concentration is out of range), further action is needed. In this case, a recommendation is provided to the farmer or any other user. Because the recommendation is based on the response of an animal's blood biomarker to the animal's feed, the recommendation is referred to as response-based nutrient recommendation. Such response-based nutrient recommendation is preferably the output of a software application. The software application may be installed on a computer system. Alternatively, the computer system may be used to access the software application through the internet and alike. Any kind of computer system could be used, including smart phones and other handheld devices.

In one embodiment of the present disclosure, a computer system provides a recommendation of supplementing drinking water of a group of animals with at least one electrolyte. By way of example, it may recommended to add NaCl and/or KCl to the drinking water. By doing so, the drinking water is supplemented with Na+, K+ and/or Cl-.

One embodiment of the present disclosure relates to a preferably computer-implemented method of supplementing the drinking water of a group of animals with at least one electrolyte, said method comprising the steps:

(i) receiving data, said data comprising the concentration of Na+, K+ and/or Cl- in the blood of at least one animal of a group of animals,

(ii) providing a model which allows to calculate the concentration of Na+, K+ and/or Cl- in drinking water that is suitable for watering an animal having a given concentration of Na+, K+ and/or Cl- in its blood,
(iii) using the data of step (i) and the model of step (ii) for supplementing the drinking water of said group of animals with Na+, K+ and/or Cl-, and

wherein said group of animals is preferably a flock of birds, and wherein said birds are preferably chicken, turkeys, ducks or geese, and wherein said chicken are preferably laying hens or broilers.

The person skilled in the art will understand that the order of step (i) and (ii) is not important. Thus, step (ii) can also done before step (i), or step (i) and step (ii) can be done simultaneously.

The animals of the group of animals receive the same feed and water of the same water supply. Thus, the animals will have blood nutrient concentrations that are somewhat similar. Accordingly, it might be sufficient to measure blood nutrient concentrations of one animal only. Preferably, however, the concentrations of Na+, K+ and/or Cl- in the blood of at least three animals of the group is measured. An average can then be calculated.

In a less preferred embodiment of the disclosure, only one blood nutrient concentration is measured (Na+, K+ or Cl-). However, in a preferred embodiment, the concentrations of Na+, K+ and Cl- are measured in the blood of at least one animals of the group. This will allow to calculate dEB.

Preferably, handheld blood analyzers or any other suitable point-of-care device has been used to measure *ex vivo* the blood concentrations of Na+, K+ and/or Cl- that are received in step (i). Preferred handheld blood analyzers provide the blood concentrations of all three ions (i.e. of Na+, K+ and Cl-). Heparinized blood (approximately 0.2 ml) can be analyzed in the i-Stat® Alinity v handheld blood analyzer fitted with a Chem8+ cartridge (Abbott Point of Care Inc., Princeton, NJ), which measures hematocrit, hemoglobin, blood urea nitrogen, creatinine, calcium, glucose, chloride, sodium, potassium, total carbon dioxide (TCO2), and anion gap (AnGap). Similarly heparinized blood (0.1 ml) can be analyzed in the Vetscan® VS2 Chemistry Analyzer (Abaxis, inc) using the Avian/Reptilina Profile Plus cartridge (Abbott Point of Care Inc., Princeton, NJ), which meaures aspartate aminotransferase, creatine kinase, uric acid, glucose, calcium, phosphorus, total protein (TP), albumin, albumin/globulin, potassium and sodium.

In case of relatively small animals, the blood of more than one animal of a group may be examined. Within one group/flock of animals, all animals are fed in the same manner and thus, an average can be calculated using measured blood nutrient concentrations from more than one animal from the same group. Thus, one embodiment relates to a computer-implemented method of supplementing drinking water of a group of animals with at least one electrolyte, wherein blood concentrations from more than one animal of the same group are received in step (i) to calculate an average, and wherein said average is then used in step (iii) This embodiment is particularly preferred for a flock of a chicken, turkeys, ducks or geese. Other than that, the herein disclosed computer-implemented method may be used for any kind of animals and in particular for monogastric animals including pigs. Nevertheless, a preferred embodiment of the disclosure relates to a computer-implemented method of supplementing drinking water of a group of animals with electrolytes, wherein the group of animals is a flock of birds, and wherein said birds are preferably chicken, turkeys, ducks or geese, and wherein said chicken are preferably laying hens or broilers.

In step (iii), drinking water is supplemented with Na+, K+ and/or Cl-. There are multiple ways how this can be done. In a preferred embodiment, an aqueous stock solution comprising Na+, K+ and/or Cl- is prepared in step (iii) This aqueous stock solution is then added to the drinking water of the group of animals. Thus, in a preferred embodiment of the computer-implemented method of supplementing drinking water of a group of animals with electrolytes, an aqueous stock solution comprising Na+, K+ and/or Cl- is prepared in step (iii), wherein said aqueous stock solution is added to the drinking water of said group of animals, and wherein said flock of animals is preferably a flock of birds.

There are various manners how an aqueous stock solution can be added to drinking water of animals. In case of birds (e.g. broilers), a medicator is preferably used to inject an aqueous stock solution comprising Na+, K+ and/or Cl- into drinking water flowing in a water flow line that is used to water the flock of birds. Thereby, said aqueous stock solution is added to the drinking water at a predetermined ratio of the aqueous stock solution to drinking water. When using a commercially available medicator, the predetermined ratio of the aqueous stock solution to drinking water is preferably from 1:80 to 1:150 and is more preferably 1:100 or 1:128.

The predetermined ratio of the aqueous stock solution to drinking water depends on various factors, including the concentration of the aqueous stock solution and the concentration of Na+, K+ and/or Cl- in the drinking water before supplementation. It is therefore preferred that the concentration of Na+, K+ and/or Cl- in the drinking water of the group of animals before supplementation is also received in step (i).

It is a benefit of the present disclosure that the concentration of the aqueous stock solution and/or the predetermined ratio of the aqueous stock solution to drinking water can be quickly adjusted, e.g. depending on the water to feed intake ratio of the birds in the shed. Birds drink usually approx. 2 liters of water per kg of feed consumed. However, the water to feed intake ratio of the birds can vary from around 1.8 to 3.5 or can even be around 1, depending on feed composition and ambient temperature. In a preferred embodiment of the disclosure, the expected water to feed intake ratio is taken into account by the model that is provide in step (ii). In this preferred embodiment, ambient air temperature of the group of animals is also received in step (i). Thus, in a preferred embodiment of the computer-implemented method of supplementing drinking water of a group of animals with at least one electrolyte, ambient air temperature of the group of animals is also received in step (i), and the model provided in step (ii) takes into account that an increase in ambient air temperature increases the average water consumption of the animals.

A particularly preferred embodiment of the disclosure relates to a computer-implemented method of supplementing the drinking water of a flock of broilers with at least one electrolyte, said method comprising the steps:

(i) receiving data, said data comprising the concentration of Na+, K+ and Cl-in the blood of at least one broiler of a flock of broilers,

(ii) providing a model which allows to calculate the concentration of Na+, K+ and Cl- in drinking water that is suitable for watering a broiler having a given concentration of Na+, K+ and Cl- in its blood,

(iii) using the data of step (i) and the model of step (ii) for supplementing the drinking water of said flock of broilers with Na+, K+ and/or Cl-,

wherein optionally step (ii) is done before step (i), or wherein optionally step (i) and step (ii) are done simultaneously, and/or

wherein the concentration of Na+, K+ and Cl- in the drinking water of the flock of broilers before supplementation is also received in step (i), and/or

wherein ambient air temperature of the flock of broilers is also received in step (i), and/or wherein the model provided in step (ii) takes into account that an increase in ambient air temperature increases the average water consumption of the broilers, and/or

wherein an aqueous stock solution comprising Na+, K+ and/or Cl- is prepared in step (iii), and wherein said aqueous stock solution is added to the drinking water of said flock of broilers, preferably using a medicator, and, wherein said aqueous stock solution is added to the drinking water of said flock of broilers at a predetermined ratio of the aqueous stock solution to drinking water, and wherein said ratio is preferably from 1:80 to 1:150 and is more preferably 1:100 or 1:128.

## 2) Use of the disclosure

The present disclosure also relates to a novel use a known point-of-care device, preferably to a novel use a handheld blood analyzer. Hitherto, point-of-care devices have never been used in a preferably computer-implemented method of supplementing drinking water of a group of animals with at least one electrolyte as herein described.

A preferred embodiment of the disclosure relates to the use of at least one point-of-care devices in a computer-implemented method of supplementing drinking water of a group of animals with at least one electrolyte, wherein said point-of-care device is suitable for measuring the concentration Na+, K+ and/or Cl- in the blood of an animal, and wherein said measurement is preferably done *ex vivo.*

A further embodiment of the disclosure relates to the use of at least one blood concentration of a biomarker for controlling an apparatus for metering aqueous stock solution into a water flow line, wherein said apparatus for metering is preferably a medicator and wherein aqueous stock solution comprises preferably Na+, K+ and/or Cl-.

A yet further embodiment of the disclosure relates to the use of a medicator for metering an aqueous stock solution comprising Na+, K+ and/or Cl- into the drinking water of the flock of animals. Hitherto, medicators have been used for pharmaceuticals. Using medicators in precision nutrition (i.e. for non-pharma purposes) for adding relatively large volumes of a stock solution is novel and surprising.

## 3) Method of treatment or prevention of respiratory alkalosis

In some cases, it is possible to lower blood levels of nutrients that have been detected to be too high. By way of example, if Na+ level in the blood is too high, one could add Cl- to the drinking water to at least achieve a better dEB. Nevertheless, it is often a lot easier and more straightforward to raise blood nutrient levels that have been detected to be too low. Therefore, it is preferred to feed animals with a diet that has a lower than normal dEB and, if needed, to add Na+, K+ and/or Cl- to the animals' drinking water. One embodiment of the present disclosure relates to the treatment or prevention of respiratory alkalosis in an animal, wherein said animal is fed with feed having a lower than normal dEB, and wherein an aqueous stock solution comprising Na+, K+ and/or Cl- is added to the animals' drinking water, if the respective blood concentrations indicate that this is needed.

One embodiment of the present disclosure relates to broiler feed for use in the treatment or prevention of respiratory alkalosis, wherein said feed has a dietary electrolyte balance (dEB) from 100 to 170 milliequivalents (mEq), preferably from 100 to 145 milliequivalents (mEq). This is a low dEB diet; most broiler feed has a dEB from 220-250 mEq. Occasionally, there is even broiler feed with >250 mEq.

There are multiple manners how feed having a dietary electrolyte balance (dEB) from 100 to 170 milliequivalents (mEq) can be provided. In standard broiler diet, there are multiple sources of chloride such choline chloride, lysine hydrochloride, betaine hydrochloride, NaCl and occasionally ammonium chloride. According to the present disclosure, it is preferred to use the diet's NaCl concentration to adjust the diet's dEB. By keeping the NaCl concentration of the diet lower than normal, feed having a lower than normal dietary electrolyte balance (dEB) is obtained. Thus, a preferred embodiment of the present disclosure relates to broiler feed for use in the treatment or prevention of respiratory alkalosis, wherein said feed has a dietary electrolyte balance (dEB) from 100 to 170 milliequivalents (mEq), preferably from 100 to 145 milliequivalents (mEq), and wherein said feed comprises less than 0.5 weight-% NaCl, preferably less than 0.2 weight-% NaCl, based on the total weight of the broiler feed. Feed having a particularly low dietary electrolyte balance (dEB) is obtained when some of the feed's NaCl is replaced by sodium carbonate and/or sodium bicarbonate. Similarly, KCl may be replaced by a potassium salt without chloride such as potassium sulphate. Thus, a preferred embodiment of the present disclosure relates to broiler feed for use in the treatment or prevention of respiratory alkalosis, wherein said feed comprises less than 0.5 weight-% NaCl, preferably less than 0.2 weight-% NaCl, based on the total weight of the broiler feed, and wherein said feed comprises sodium carbonate, sodium bicarbonate and/or potassium sulphate.

The present disclosure also relates to the use of the herein disclosed broiler feed for raising a flock of broilers. A preferred embodiment relates to a method of raising a flock of broilers, wherein broiler feed having a dietary electrolyte balance (dEB) from 100 to 170 milliequivalents (mEq), preferably from 100 to 145 milliequivalents (mEq), is fed to the broilers, and wherein an aqueous stock solution comprising Na+, K+ and/or Cl- is added to the drinking water of the broilers. Thereby, blood nutrient concentrations are used to determine the amount of Na+, K+ and/or Cl- that needs to be added to the drinking water. Adding Na+, K+ and/or Cl- to the drinking water of the broilers does not exclude the possibility of altering the dEB in the feed as an additional (although much slower) mediation strategy.

*4. Use of Drinking Water as a Medium to Supply Nutrients in an Amount That Precisely Meets the Nutritional Requirements of Animals to be Raised*

The general remarks of or the embodiments in the previous sections and Figs. 1-9 may also apply here, but for conciseness they are not reiterated.

A "set-up" is a way in which things are arranged. In a preferred embodiment of the disclosure, the set-up comprises a computer system, at least one point-of-care device at least one water flow line and at least one apparatus for metering nutrients into a water flow line. In a preferred embodiment of the disclosure, some or all of these three elements are operatively linked. By way of example, the point-of-care device may send data to the computer system and/or may receive data from the computer system, whereas the computer system may send data to the apparatus for metering. These three elements may be located on a farm where animals are raised. The set-up of the disclosure may comprise further elements, e.g. a stock of nutrients.

The apparatus for metering nutrients is preferably a medicator. A "medicator" is an apparatus (preferably an injector) for introducing a drug or other substance into a liquid supply line in controlled amounts. Preferred medicators withdraw a set amount of a solution (e.g. water comprising electrolytes) and injects it into the water lines of the farm. The target animals then consume the solution over time. Thereby, the solution to water ratio can be adjusted. A typically ratio may be 1:128, i.e. 1 oz. of solution to 128 oz. (1 gal.) of water.

In the context of the present disclosure, a group of animals are animals of a given species that receive the same feed, the same drinking water and are exposed to the same ambient air temperature. A "flock" is a group of animals comprising preferably at least 10, more preferably to at least 100 and most preferably to at least 1000 animals that are raised in the same compartment (e.g. in the same pen or in the same house). In a flock of animals, the animals have approximatively the same age. In case of birds, "approximatively the same age" means that age may vary by 1-2 days because batches of eggs put in the incubator will hatch from around 20-22 days after placement.

**1) Computer-implemented method of raising a flock of animals**

Over a relatively large range, the amount of ingested nutrient correlates with the observed blood nutrient concentration: the more of nutrient an animal ingests, the higher will be the concentration of the nutrient (or of a metabolite thereof) in the animal's blood. Provided sufficient data is available for calibration, the person skilled in the art can find values for parameters needed for a suitable mathematical prediction model. If the nutritional log of the animal is available, it is particularly straightforward to predict the concentration of a nutrient in the blood of an animal.

If the measured blood nutrient concentration matches the predicted blood nutrient concentration (i.e. if the measured blood nutrient concentration is in range), everything seems fine. Most likely, the actual feed nutrient concentration matches the nominal feed nutrient concentration and most likely, the animal's bioavailability is as it should be. Thus, there is no need to alter the current situation on farm.

However, if the measured blood nutrient concentration is below the expected range, further action is needed.

Thus, a recommendation may be provided to the farmer or any other user. Preferably, the recommendation is to add nutrient to the drinking water of the respective animals. Such response-based nutrient recommendation is preferably the output of a software application. The software application is preferably installed on a computer system.

One embodiment of the present disclosure relates a computer-implemented method of raising a flock of animals, said method comprising the steps:

(i) receiving a blood nutrient concentration of an animal of the flock, wherein said blood nutrient concentration has been measured *ex vivo,* and further receiving the nutritional log of the animal whose blood has been examined *ex vivo,*

(ii) providing a model which defines blood nutrient concentrations that are in range if the animal whose blood is examined *ex vivo* has been fed for a predetermined period of time with a predetermined amount of feed, said feed comprising the corresponding nutrient in a given concentration,

(iii) using the model of step (ii) to determine if the blood nutrient concentration received in step (i) is in range, below range or above range, and

(iv) if step (iii) reveals that the blood nutrient concentration received in step (i) is below range: recommending the addition of the nutrient to the drinking water of the flock of animals.

There are various manner how nutrients can be added to drinking water of animals. Preferably, a medicator is used to inject a typically aqueous composition comprising the nutrient into drinking water flowing in a water flow line that is used to water the flock of animals. Thus, in a preferred embodiment, the blood nutrient concentration received in step (i) is the blood nutrient concentration of a nutrient that can be metered into drinking water of animals.

The composition to be injected by the medicator is preferably an aqueous composition. In case of water-soluble nutrients, said aqueous composition may by a solution. This applies e.g. to water-soluble salts containing water-soluble ions. Preferred ions are sodium, potassium, chloride, phosphorus and calcium. In case of a water-dispersible nutrients, the composition comprising the nutrient may be an aqueous dispersion. In case of fat-soluble nutrients, the composition comprises preferably a water-soluble or water-dispersible formulation of the nutrient. Such formulations are commercially available as DSM® Nutritional Products, Switzerland. An aqueous composition comprising such water-soluble or water-dispersible formulation of the nutrient may then be injected into to the drinking water of the respective animals. Thus, in step (iv) of the computer-implemented method of raising a flock of animals, the recommendation is preferably given to use a medicator to inject a composition comprising the nutrient into drinking water flowing in a water flow line that is used to water the flock of animals.

Many handheld blood analyzers provide blood concentrations of more than one nutrient. Depending on the type of blood analyzer, concentrations of sodium, potassium, chloride, phosphorus and calcium may be provided in one go by measuring one blood sample only. Thus, one embodiment relates to a computer-implemented method of raising a flock of animals, wherein more than one blood nutrient concentration is received in step (i), and wherein the addition of more than one nutrient is recommended in step (iv) if step (iii) reveals that more than one blood nutrient concentration is below range.

In case of relatively small animals, the blood of more than one animal of a flock may be examined. Within one flock, all animals are fed in the same manner and thus, an average can be calculated using measured blood nutrient concentrations from more than one animal from the same flock. Thus, one embodiment relates to a computer-implemented method of raising a flock of animals, wherein concentrations from more than one animal of the same flock are received in step (i) to calculate an average, and wherein said average is then used in step (iii). This embodiment is particularly preferred for a flock of a chicken, turkeys, ducks or geese. Other than that, the herein disclosed computer-implemented method of raising a flock of animals may be used for any animal and in particular for monogastric animals such as pigs.

The person skilled in the art will understand that the order of step (i) and (ii) is not important. Thus, step (ii) could done before step (i), or step (i) and step (ii) could be done simultaneously.

## 2) Set-up for raising animals

To practice the herein disclosed a computer-implemented method of raising a flock of animals, the set-up of the present information disclosure is preferably used. Preferably, the set-up of the disclosure is a set-up for raising animals comprising:

a) at least one computer system,
b) at least one point-of-care device, and
c) at least one water flow line and at least one apparatus for metering nutrients into said water flow line, and

wherein said at least one point-of-care device is preferably a handheld blood analyzer, and
wherein said at least one apparatus for metering nutrients is preferably a medicator.

According the disclosure, a model is used to determine if the blood nutrient concentration provided by the handheld blood analyzer is in range, below range or above range. Such model is preferably part of a computer program that is stored on a computer system. Any kind of computer system can be used as long as it has access to a model which defines blood nutrient concentrations of an animal that are in range if the animal has been fed for a predetermined period of time with a predetermined amount of feed, said feed comprising nutrients in a given concentration. One embodiment of the present disclosure relates to a set-up for raising animals, wherein said computer system is a desktop, laptop or smartphone.

The set-up of the disclosure is suitable for raising animals that need to be watered. Accordingly, the preferred medicator is capable of injecting an aqueous composition into a water flow line that is used for watering the animals to be raised.

Any metering device needs to be told how much of a composition needs to be provided. These instructions are usually given by the user of the device by setting a valve or alike to a certain position. In one embodiment of the disclosure, the user is setting a valve of alike manually (e.g. electronically by pushing a button) on the basis of the measured blood nutrient concentrations as provided by the at least one point-of-care device and as interpreted by the at least one computer system. Thus, one embodiment of the present disclosure relates to a set-up, wherein said point-of-care device is a handheld blood analyzer that is suitable for measuring *ex vivo* at least one blood nutrient concentration, and wherein said handheld blood analyzer is preferably suitable for measuring *ex vivo* the concentration of at least one ion in blood of the animals to be raised, said ion being preferably sodium, potassium, chloride, phosphorus and/or calcium.

In a preferred embodiment, however, a manual intervention by the user is no longer needed. By way of example, the point-of-care device of the set-up may transmit the measured blood nutrient concentrations to the computer system of the set-up. The computer system uses the measured blood nutrient concentrations as input for the above mentioned model. The model then reveals if any nutrient should be added to the animal's drinking water (output). The computer system may then transmit data to the medicator's valve or to an electric motor that is moving the medicator's valve. Thus, a preferred embodiment of the present disclosure relates to a set-up, wherein the computer system is operatively linked to said point-of-care device, and/or wherein said computer system is operatively linked to said apparatus for metering a nutrient into a water flow line.

### 3) Use of the disclosure

The present disclosure also relates to a novel use a known point-of-care device, preferably to a novel use a handheld blood analyzer. Hitherto, blood nutrient concentrations that have been measured *ex vivo* have never be used to control a medicator (as described above).

One embodiment of the disclosure relates to the use of at least one blood concentration of a nutrient for controlling an apparatus for metering a nutrient into a water flow line, preferably for controlling a medicator (e.g. controlling at least one valve of a medicator). A further embodiment of the disclosure relates to the use of at least one blood concentration of a nutrient for metering said nutrient into drinking water of a flock of animals, wherein said blood concentration has been measured *ex vivo* in the blood of an animal of the flock.

A preferred embodiment of the disclosure relates to the use of a medicator for metering at least one nutrient into the drinking water of the flock of animals, and wherein more of the nutrient is metered into the drinking water if the blood concentration of a nutrient is below a pre-determined range. To control a valve or alike of the apparatus for metering a nutrient into a water flow line, it is preferred that the blood concentration of the nutrient has been measured *ex vivo* with a point-of-care device, wherein said point-of-care device is preferably operatively linked to a medicator.

An embodiment of the disclosure relates to the use of drinking water as a medium to supply nutrients to a flock of animals, wherein at least one nutrient is added to the drinking of the flock of animals, and wherein the amount of said at least one nutrient that is added to said drinking water is adapted to the nutritional requirements of the animals of said flock, and wherein the concentration of at least one nutrient in the blood of at least one animal of the flock has been measured *ex vivo* to determine the nutritional requirements of the animals of said flock, and wherein said measurement has preferably be done with a point-of-care device, and wherein a medicator is used for adding said at least one nutrient to the drinking of said flock of animals.

### EXAMPLES

Example 1 (calcium and phosphorus - calibration)

In example 1, broiler feed was prepared. The composition of the prepared starter diets is shown in below Table 1:

Table 1

| Ingredient | High calcium, standard phosphorus diet (weight-%, based on the total weight of the feed) | Low calcium, standard phosphorus diet (weight %, based on the total weight of the feed) | High calcium, low phosphorus diet (weight-%, based on the total weight of the feed) | Low calcium, low phosphorus diet (weight %, based on the total weight of the feed) |
|---|---|---|---|---|
| Corn | 54.28 | 58.42 | 57.37 | 60.39 |
| Soybean meal (48% CP) | 36.60 | 35.93 | 36.10 | 35.62 |
| Soy oil | 2.97 | 1.56 | 1.92 | 0.89 |
| Dicalcium phosphate (18.5% P) | 1.71 | 1.71 | 0.82 | 0.82 |
| Limestone | 2.81 | 0.74 | 2.16 | 0.64 |
| Salt | 0.28 | 0.28 | 0.28 | 0.28 |
| Sodium Bicarbonate | 0.20 | 0.20 | 0.20 | 0.20 |
| Vitamin and mineral premix[1] | 0.25 | 0.25 | 0.25 | 0.25 |
| DL-Methionine | 0.40 | 0.40 | 0.40 | 0.40 |
| Threonine | 0.17 | 0.17 | 0.17 | 0.17 |
| L-Lysine | 0.31 | 0.32 | 0.32 | 0.32 |
| Phytase | 0.02 | 0.02 | 0.02 | 0.02 |
| Corn | 54.28 | 58.42 | 57.37 | 60.39 |
| Soybean meal (48% CP) | 36.60 | 35.93 | 36.10 | 35.62 |
| Soy oil | 2.97 | 1.56 | 1.92 | 0.89 |
| **Total** | **100** | **100** | **100** | **100** |

[1]Vitamin premix supplied the following per kg of diet: 13,228 IU vitamin A, 3,968 IU vitamin $D_3$, 66 IU vitamin E, 0.04 mg vitamin $B_{12}$, 0.25 mg biotin, 4 mg menadione ($K_3$), 4 mg thiamine, 13 mg riboflavin, 22 mg d-pantothenic acid, 8 mg vitamin $B_6$, 110 mg niacin, and 2.2 mg folic acid, manganese, 60 mg; zinc, 60 mg; iron, 40 mg; copper, 5 mg; iodine, 1.5 mg; and cobalt, 0.5 mg.

The nominal nutrient concentration of the prepared feed was calculated as shown in below Table 2:

Table 2

| nutrient | High calcium, standard phosphorus diet (calculated) | Low calcium, standard phosphorus diet (calculated) | High calcium, low phosphorus diet (calculated) | Low calcium, low phosphorus diet (calculated) |
|---|---|---|---|---|
| Crude protein (wt.-%) | 21.5 | 21.5 | 21.5 | 21.5 |
| **Calcium (wt.-%)** | **1.67** | **0.84** | **1.20** | **0.60** |
| **Total phosphorus (wt. %)** | **0.65** | **0.65** | **0.50** | **0.50** |
| Available phosphorus (Wt.-%) | 0.48 | 0.48 | 0.35 | 0.35 |
| Total lysine (wt.-%) | 1.44 | 1.43 | 1.43 | 1.43 |
| Total methionine + cysteine (wt.-%) | 1.03 | 1.03 | 1.03 | 1.03 |
| Sodium (wt.-%) | 0.18 | 0.18 | 0.18 | 0.18 |
| Chloride (wt.-%) | 0.27 | 0.28 | 0.27 | 0.28 |

(continued)

| nutrient | High calcium, standard phosphorus diet (calculated) | Low calcium, standard phosphorus diet (calculated) | High calcium, low phosphorus diet (calculated) | Low calcium, low phosphorus diet (calculated) |
|---|---|---|---|---|
| Metabolizable energy (kcal/g) | 3,000 | 3,000 | 3,000 | 3,000 |
| All wt.-% on the basis of the total weight of the feed | | | | |

To make sure that the nominal, calculated calcium, and phosphorus concentration in the feed corresponds to the actual calcium and phosphorus concentration in the feed, a conventional feed assay was done in the laboratory (AOAC Official Method 975.03B(b) Metals in Plants and Pet Foods Atomic Absorption Spectrophotometric Method (applicable to Ca, Cu, K, Mg, Mn, and Zn) nitric-perchloric wet ash sample preparation with hydrochloric acid sample matrix). The feed assay confirmed that nominal calcium and phosphorus concentration corresponds to the actual calcium and phosphorus concentration.

The prepared feed was mixed as large batches of 4 diets. To create the experimental diets, each large batch of feed was split, and mixed different ratios as shown in below Table 3:

Table 3

| Diet | Percent of high calcium, standard phosphorus diet | Percent of low calcium, standard phosphorus diet | Percent of high calcium, low phosphorus diet | Percent of low calcium, low phosphorus diet |
|---|---|---|---|---|
| 1 | 100 | 0 | 0 | 0 |
| 2 | 67 | 33 | 0 | 0 |
| 3 | 33 | 67 | 0 | 0 |
| 4 | 0 | 100 | 0 | 0 |
| 5 | 0 | 0 | 100 | 0 |
| 6 | 0 | 0 | 67 | 33 |
| 7 | 0 | 0 | 33 | 67 |
| 8 | 0 | 0 | 0 | 100 |

This created eight experimental diets containing graded levels of calcium and two levels of phosphorus as shown in below Table 4:

Table 4

| Diet | Phosphorus, % | Calcium, % |
|---|---|---|
| 1 | | 1.67 |
| 2 | 0.65 | 1.39 |
| 3 | | 1.11 |
| 4 | | 0.84 |
| 5 | | 1.20 |
| 6 | 0.50 | 1.01 |
| 7 | | 0.80 |
| 8 | | 0.60 |

Each prepared experimental diet was fed to 12 pens of 27 birds (Ross 308 broilers) each, starting from hatch and ending when the birds reached 12 days of age. When the birds reached 12 days of age, the calcium and phosphorus concentration was determined in the venous blood of 1 bird per pen of average body weight, using a commercially available point-of-care device: Vetscan® VS2 Chemistry Analyzer (Abaxis, Inc) using the Avian/Reptilian Profile Plus cartridge (Abbott Point of Care Inc., Princeton, NJ).

Fig. 4A summarizes the calcium results of example 1. It shows that calcium in the blood (mmol/L) decreases (quadratic, $P < 0.05$) as the total calcium in the diet (weight-%) decreases from 1.67 to 0.60%. The relationship between the calcium in the prepared diets and the plasma calcium concentration can also be shown as depicted in Fig. 4B (amount of calcium on the x-axis increasing from left to right, opposite x-axis of Fig. 4A). Regression curves are shown in both figures, which may be used to generate a parameterized model as described elsewhere in this specification. Fig. 4C shows plasma phosphorus (mmol/L) increases ($P < 0.05$) as total phosphorus in the diet increases from 0.50 to 0.65%.

Example 1 shows how blood biomarkers respond to a variation in nutrient concentration in feed: If the actual concentration of a nutrient in the provided feed is too low, the value for the corresponding blood biomarker will drop. Thus, after calibration, e.g., by regression analysis, doing a conventional feed assay is no longer necessary: if the actual nutrient concentration in the feed is for any reason below the calculated nutrient concentration in the feed, such discrepancy will be detected by the biomarkers' response to the ingested feed. This is faster and cheaper than wet chemistry analysis of feed. If both is done, conventional feed assay and blood analysis, the information from the conventional feed assay is augmented: If the values for a blood biomarker is low although conventional feed assay shows that the calculated nutrient concentration in the feed corresponds to the actual nutrient concentration in the feed, the cause for the too low values has been found: decreased bioavailability of the respective nutrient. Adequate measures can be taken. Summarizing, example 1 shows that feed mixing and/or quality errors can be detected very fast and cost-effective when practising the herein disclosed disclosure.

Example 2 (sodium ions - calibration)

In example 2, four broiler feeds were prepared: 2 variants of a starter diet; and 2 variants of a grower diet. Starter diets have a higher protein content than grower diets.

The feeds of example 2 were based on corn and soybean meal and were formulated to meet the nutrient requirements of the birds for all macro- and micro-nutrients. One variant of feed was formulated using sodium chloride (NaCl; noted as "Salt") whereas the other feed variant was formulated using a combination of sodium chloride (NaCl) and sodium bicarbonate ($NaHCO_3$; noted as "Bi-Carb"). This was done to reflect industry practises in provision of sodium and chloride to growing birds.

The compositions of the four broiler feeds prepared in example 2 are shown in below Table 5:

Table 5

| | Starter[4] | | Grower[5] | |
|---|---|---|---|---|
| | Salt | Bi-Carb | Salt | Bi-Carb |
| Ingredient (wt.-%) | | | | |
| Corn | 58.25 | 58.22 | 65.45 | 65.41 |
| Soybean meal (48% CP) | 33.81 | 33.81 | 26.72 | 26.70 |
| Distillers Dried Grains | 2.00 | 2.00 | 2.0 | 2.0 |
| Poultry by-product meal | 2.00 | 2.00 | 2.0 | 2.0 |
| Poultry fat | 0.50 | 0.50 | 0.50 | 0.50 |
| Dicalcium phosphate (18.5% P) | 1.53 | 1.53 | 1.3 | 1.3 |
| Limestone | 1.05 | 1.05 | 0.95 | 0.95 |
| **Salt** | **0.40** | **0.28** | **0.44** | **0.3** |
| **Sodium Bicarbonate** | **0.00** | **0.18** | | **0.2** |
| Vitamin premix[1] | 0.05 | 0.05 | 0.05 | 0.05 |
| Mineral premix[2] | 0.20 | 0.20 | 0.20 | 0.20 |
| Rovimix C-EC | | | | |
| Rovimix E 50% | | | | |
| Selenium premix[3] | 0.05 | 0.05 | 0.05 | 0.05 |
| DL-Methionine | 0.07 | 0.07 | 0.19 | 0.19 |

(continued)

|  | Starter[4] | | Grower[5] | |
| --- | --- | --- | --- | --- |
|  | Salt | Bi-Carb | Salt | Bi-Carb |
| Ingredient (wt.-%) | | | | |
| L-Lysine | 0.08 | 0.08 | 0.15 | 0.15 |
| Total | 100 | 100 | 100 | 100 |
|  | | | | |
| Calculated nutrient concentration (wt.-% unless otherwise stated) | | | | |
| Crude protein | 22.5 | 22.5 | 20.0 | 20.0 |
| Calcium | 0.90 | 0.90 | 0.80 | 0.8 |
| Available phosphorus | 0.45 | 0.45 | 0.40 | 0.40 |
| Potassium | 0.85 | 0.85 | 0.74 | 0.74 |
| Total lysine | 1.31 | 1.31 | 1.17 | 1.17 |
| Total methionine | 0.44 | 0.44 | 0.52 | 0.52 |
| Total threonine | 0.90 | 0.90 | 0.78 | 0.78 |
| Total methionine + cysteine | 0.74 | 0.74 | 0.78 | 0.78 |
| **Sodium** | **0.20** | **0.20** | **0.21** | **0.21** |
| Chloride | 0.29 | 0.22 | 0.31 | 0.23 |
| Metabolizable energy (kcal/g) | 2,998 | 2,996 | 3,070 | 3,068 |
| Dietary Electrolyte Balance (meq/kg) | 223 | 244 | 193 | 216 |
|  | | | | |
| Analyzed nutrient concentration | | | | |
| Crude Protein | 23.15 | 23.38 | 20.23 | 20.93 |
| Crude Fat | 3.54 | 3.14 | 3.80 | 3.15 |
| Vitamin E(mg/kg) | 7 | 6 | 6 | 5 |
| Vitamin C (mg/kg) | . | 29 | 16 | 18 |
| CRINA (mg/kg) | . | . | . | . |
| **Sodium** | **0.19** | **0.21** | **0.23** | **0.22** |
| Chloride | 0.27 | 0.21 | 0.29 | 0.23 |
| Potassium | 1.122 | 1.109 | 0.953 | 0.997 |
| Dietary Electrolyte Balance (meq/kg) | 294 | 317 | 263 | 287 |

[1]Vitamin premix supplied the following per kg of diet: 13,200 IU vitamin A, 4,000 IU vitamin $D_3$, 33 IU vitamin E, 0.02 mg vitamin $B_{12}$, 0.13 mg biotin, 2 mg menadione ($K_3$), 2 mg thiamine, 6.6 mg riboflavin, 11 mg d-pantothenic acid, 4 mg vitamin $B_6$, 55 mg niacin, and 1.1 mg folic acid.
[2]Mineral premix supplied the following per kg of diet: manganese, 120 mg; zinc, 120 mg; iron, 80 mg; copper, 10 mg; iodine, 2.5 mg; and cobalt, 1 mg.
[3]Selenium premix provided 0.2 mg Se (as $Na_2SeO_3$) per kg of diet.
[4]Starter diet was fed to approximately 14 d of age, 910 g per bird.
[5]Grower diet was fed from approximately 15 to 28 d of age, 2,750 g per bird.

To make sure that the calculated $Na^+$ concentration in the diets corresponds to the actual $Na^+$ concentration in the feed, a conventional feed assay was done in the laboratory. The feed assays confirmed that in all four diets, calculated $Na^+$ concentration (cf. "analyzed nutrient concentration" in above table) corresponds within reason to the actual $Na^+$ concentration (cf. "analyzed nutrient concentration" in above table). The four diets were then fed to 8 replicate pens of 20 (10 male and 10 female) Hubbard-Cobb broilers. The two starter diets were fed from d1-14 whereas the two grower diets were fed to the same broilers from d15-28. Temperature and lighting were controlled as per breed guidelines and within normal ranges for commercial broiler production. Live performance was monitored throughout the experiment and venous blood was taken on d28 analyzed for Na+ using the i-Stat® Alinity v handheld blood analyzer fitted with a Chem8+ cartridge (Abbott Point of Care Inc., Princeton, NJ). At d28, Na+ concentration of approx. 143 mmol per liter blood was measured, regardless of whether sodium chloride ("Salt") was the only source of Na+ or whether a combination of salt chloride and sodium bicarbonate ("Bi-Carb") was used as a source of Na+.

Example 3 (Sodium ions - feed issue)

In example 3, two variants of a finisher diet were prepared, similar to example 2. The compositions of the two variants of finisher diet are shown in below Table 6:

Table 6

|  | Finisher[6] | |
| --- | --- | --- |
|  | Salt | Bi-Carb |
| Ingredient (wt.-%) |  |  |
| Corn | 71.59 | 71.39 |
| Soybean meal (48% CP) | 20.70 | 20.72 |
| Distillers Dried Grains | 2.0 | 2.0 |
| Poultry by-product meal | 2.0 | 2.0 |
| Poultry fat | 0.70 | 0.78 |
| Dicalcium phosphate (18.5% P) | 1.2 | 1.21 |
| Limestone | 0.91 | 0.91 |
| **Salt** | **0.49** | **0.30** |
| **Sodium Bicarbonate** |  | **0.28** |
| Vitamin premix[1] | 0.05 | 0.05 |
| Mineral premix[2] | 0.20 | 0.20 |
| Rovimix C-EC |  |  |
| Rovimix E 50% |  |  |
| Selenium premix[3] | 0.05 | 0.05 |
| DL-Methionine | 0.08 | 0.08 |
| L-Lysine | 0.03 | 0.03 |
| Total | 100 | 100 |
|  |  |  |
| Calculated nutrient concentration (wt.-% unless otherwise stated) |  |  |
| Crude protein | 17.50 | 17.5 |
| Calcium | 0.75 | 0.75 |
| Available phosphorus | 0.38 | 0.38 |
| Potassium | 0.65 | 0.65 |
| Total lysine | 0.90 | 0.90 |
| Total methionine | 0.38 | 0.38 |
| Total threonine | 0.68 | 0.68 |
| Total methionine + cysteine | 0.61 | 0.61 |
| **Sodium** | **0.23** | **0.23** |

(continued)

| Calculated nutrient concentration (wt.-% unless otherwise stated) | | |
|---|---|---|
| Chloride | 0.35 | 0.23 |
| Metabolizable energy (kcal/g) | 3,150 | 3150 |
| Dietary Electrolyte Balance (meq/kg) | 167 | 200 |
| | | |
| [1]Vitamin premix supplied the following per kg of diet: 13,200 IU vitamin A, 4,000 IU vitamin $D_3$, 33 IU vitamin E, 0.02 mg vitamin $B_{12}$, 0.13 mg biotin, 2 mg menadione ($K_3$), 2 mg thiamine, 6.6 mg riboflavin, 11 mg d-pantothenic acid, 4 mg vitamin $B_6$, 55 mg niacin, and 1.1 mg folic acid. | | |
| | Finisher[6] | |
| | Salt | Bi-Carb |
| [2]Mineral premix supplied the following per kg of diet: manganese, 120 mg; zinc, 120 mg; iron, 80 mg; copper, 10 mg; iodine, 2.5 mg; and cobalt, 1 mg.<br>[3]Selenium premix provided 0.2 mg Se (as $Na_2SeO_3$) per kg of diet.<br>[6]Finisher diet was fed from approximately 28 to end of experiment. | | |

The prepared feed was fed to broilers starting when the birds reached 28 days of age. The same flock as in example 1 was used. Thus, the broilers of example 2 were switched to the feed of example 2a at the age of 28 days. Venous blood from 2 male broilers per pen were analyzed at day 42 using the i-STAT® Handheld Clinical Analyzer.

Example 3 (finisher diet) is the continuation of example 2 (grower diet, same birds, same location). The i-STAT® data from example 3 can therefore be compared with the i-STAT® data from example 2. In example 2, a sodium concentration of approx. 0.21 weight-%, based on the total weight of the feed, resulted in more than 143,5 mmol sodium per liter blood (cf. Fig. 4D, left side). In the finisher diet of example 3, the sodium concentration has been raised to approx. 0.23 weight-%, based on the total weight of the feed (cf. Table 6). Thus, the blood samples of example 3 were expected to also contain more than 143,5 mmol sodium per liter blood. Surprisingly, this was not the case. At day 42, Na+ concentrations of 142 mmol per liter blood ("Salt" variant of finisher feed) and 143.2 mmol per liter blood ("Bi-Carb" variant of finisher feed) were measured. Thus, for both variants' finisher feed, a lower-than-expected blood concentration of Na+ was measured (cf. Fig. 4D, right side). The issue was resolved by a wet chemistry analysis of the two variants of the finisher feed of example 3. The result is shown in below Table 7:

Table 7

| | Finisher[6] | |
|---|---|---|
| | Salt | Bi-Carb |
| | | |
| Analyzed nutrient concentration (wt.-% unless otherwise stated) | | |
| Crude Protein | 19.00 | 19.06 |
| Crude Fat | 5.50 | 4.34 |
| Vitamin E(mg/kg) | 113 | 105 |
| Vitamin C (mg/kg) | . | . |
| CRINA (mg/kg) | . | . |
| **Sodium** | **0.16** | **0.18** |
| Chloride | 0.24 | 0.18 |
| Potassium | 0.949 | 0.9362 |
| Dietary Electrolyte Balance (meq/kg) | 245 | 268 |
| [6]Finisher diet was fed from approximately 28 to end of experiment. | | |

For both variants of the finisher feed, the measured concentrations were lower than calculated concentrations (0.16 wt.-% and 0.18 wt.-% instead of the calculated 0.23 wt.-%; Tables 6 and 7). In other words, the calculated concentration proved to be a theoretical value only that did not reflect reality. This discrepancy explains the unexpectedly low sodium blood concentrations that were measured at day 42. At day 42, the broilers might have been underperforming. Example 3 shows how the reason for the possible underperformance was detected by measuring blood concentrations: the broilers did not get enough Na+. Wet chemistry methods showed that the examined sample of finisher feed did not contain enough Na+. If blood concentrations had been in the expected ranges, no wet chemistry analysis would have been done. Omitting routine wet chemistry analysis saves time and cost.

Example 4 (Sodium ions - mild diarrhea)

In example 4, Na+ concentrations lower than expected are measured in the blood of broilers, similar to example 3. In example 4, however, wet chemistry analysis shows that the Na+ concentration in the feed is correct (e.g., as calculated). As a consequence, a closer look is taken at the broiler's litter. Some wet droppings (chicken diarrhea) are observed. Further investigations reveal that rancid fat has been fed which led to the observed mild diarrhea. Adequate measures are taken.

Example 5 (Sodium ions - cost savings)

In example 5, an i-Stat® Alinity v handheld blood analyzer fitted with a Chem8+ cartridge is used to measure Na+ concentrations in the blood of broilers, similar to the previous example. The measured concentrations are in the expected ranges. Therefore, no standard assay is done. Standard essays in a specialized lab are significantly more expensive than using a commercially available handheld device on the farm. Thus, significant cost was saved in example 5.

Example 6A (vitamin C - time gain)

In example 6A, blood samples of broilers are analyzed with a handheld device. This can done be on the spot, e.g., on the farm. The result of the analysis is obtained within less than 2 minutes. The vitamin C blood concentrations are lower than expected. As a consequence, vitamin C is added to the drinking water of the broilers.

Wet chemistry in a lab would have taken 1-2 days. Valuable time is gained by doing on the spot blood sampling with a commercially available handheld device.

Example 6B (Chloride - time gain)

In example 6B, blood samples of broilers of a flock are analyzed with a handheld device. This can done be on the spot, i.e. on the farm. The result of the analysis is obtained within less than 2 minutes. The chloride blood concentrations are lower than expected. As a consequence, an aqueous composition Cl- is added to the drinking water of the flock of broilers. This can be easily achieved by using a medicator. Suitable medicators are commercially available e.g. at Dosatron International, LLC. The Dosatron D128R® medicator is preferred. It is a water-powered medicator using the water from the main line. This model is compatible with electrolytes with ratio range settings of 1:128, 1:100, and 1:200.

Example 7 (Chloride - comparative example)

In example 7, blood samples of broilers of a flock are analyzed with a handheld device. This can done be on the spot, i.e. on the farm. The result of the analysis is obtained within less than 2 minutes. The chloride blood concentrations are lower than expected. As a consequence, it is decided to modify the broilers feed by adding more chloride. Unfortunately, a large amount of feed as already be prepared. Thus, the feed for the current flock cannot be modified. The learnings from the analysis of chloride blood concentrations will be implemented for a future flock.

Examples, embodiments or optional features, whether indicated as non-limiting or not, are not to be understood as limiting the disclosure as claimed. For the sake of clarity, the invention is defined and solely limited by the claims.

It should be noted that the above-mentioned embodiments illustrate rather than limit the disclosure, and that those skilled in the art will be able to design many alternative embodiments without departing from the scope of the appended claims.

In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of" when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The disclosure may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A method for feed surveillance comprising:

   measuring blood nutrient concentrations ex vivo for a plurality of animals receiving data comprising said blood nutrient concentrations;
   receiving a nutritional log comprising nominal feed nutrient concentrations in a feed of the plurality of animals;
   determining, based on the data, and using a machine learning model, deduced feed nutrient concentrations in the feed of the plurality of animals, wherein the deduced feed nutrient concentrations are associated with concentrations of a plurality of (micro-)nutrient biomarkers indicative of a nutritional state of the plurality of animals;
   comparing the deduced feed nutrient concentrations with the corresponding nominal feed nutrient concentrations;
   based on the comparison, determining one or more discrepancies between the deduced feed nutrient concentrations and the corresponding nominal feed nutrient concentrations; and
   after determining the one or more discrepancies, changing a feed composition to address an issue associated with the one or more discrepancies in the feed.

2. The method of claim 1, wherein the plurality of (micro-)nutrient biomarkers indicative of the nutritional state comprise biomarkers associated with an ion, a vitamin, a carotenoid, a total protein content and myo-inositol.

3. The method of claim 2, wherein the ion is selected from the group consisting of sodium, potassium, chloride, phosphorus and calcium, and wherein the iron is not a trace mineral.

4. The method according to any one of claims 2 or 3, wherein the vitamin is selected from the group consisting of vitamin A, vitamin C, vitamin D and vitamin E.

5. The method according to any one of claims 1 to 4, wherein nutrients and micronutrients whose blood concentrations are capped due to post absorptive changes, hormonal and/or transient responses are excluded from the plurality of (micro-)nutrient biomarkers, and wherein amino acids, carbohydrates, starches, fats and lipids are excluded from the plurality of (micro-)nutrient biomarkers.

6. The method according to any one of claims 1 to 5, further comprising:

   prior to changing the feed composition, determining actual feed nutrient concentrations in the feed using a wet chemistry method;
   determining additional discrepancies between the actual feed nutrient concentrations and the corresponding nominal feed nutrient concentrations;
   after determining the additional discrepancies, changing the feed composition to address an issue associated with the additional discrepancies in the feed; and
   after determining there is no discrepancy between the actual feed nutrient concentrations and the corresponding nominal feed nutrient concentrations, recommending a measure to increase a nutrient's bioavailability in the plurality of animals.

7. The method according to any one of claims 1 to 6, further comprising:
   training, based on historical data comprising blood nutrient concentrations associated with a set of animals and historic nutritional log for the set of animals, the machine learning model.

8. The method according to any one of claims 1 to 7, wherein the plurality of animals are from a same flock.

9. The method according to any one of claims 1 to 8, wherein the plurality of animals are monogastric animals, comprising a bird or pig, and the plurality of animals are most preferably a chicken, a turkey, a duck or a goose, and wherein the chicken is preferably a broiler.

10. The method according to any one of claims 1 to 9, wherein receiving the data comprises receiving the data comprising blood nutrient concentrations that have been measured from at least one point-of-care device.

11. The method according to any one of claims 1 to 10, wherein the plurality of (micro-) nutrient biomarkers are indicative of an amount of at least one (micro-)nutrient that an animal has ingested.

12. A method for feed surveillance comprising:

measuring biomarker nutrient concentrations ex vivo for a plurality of animals receiving data comprising said biomarker nutrient concentrations;
receiving a nutritional log comprising nominal feed nutrient concentrations in a feed of the plurality of animals;
determining, based on the nutritional log, and using a machine learning model, deduced biomarker nutrient concentrations in the plurality of animals, wherein the deduced biomarker nutrient concentrations are associated with nominal feed nutrient concentrations in a feed of the plurality of animals;
comparing the deduced biomarker nutrient concentrations with the corresponding measured biomarker feed nutrient concentrations;
based on the comparison, determining one or more discrepancies between the deduced biomarker nutrient concentrations and the corresponding measured biomarker nutrient concentrations; and
after determining the one or more discrepancies, changing a feed composition to address an issue associated with the one or more discrepancies.

13. A system for detecting a deficiency in a nutritional state of at least one animal, the system comprising at least a computer system (100) comprising:

at least one point-of-care device (20), configured for ex-vivo measurement of a sample of blood, wherein the blood measurement data is of at least one animal;
and at least a computer system (100) comprising :

an input interface subsystem (120) configured to access blood measurement data (22) generated by at least one point-of-care device (20),;
a processor subsystem (160) configured to:

determine, from the blood measurement data, a measured concentration of a biomarker of a feed nutrient in feed of the at least one animal;
provide a machine learning model (142) configured to model a relationship between concentrations of the biomarker and concentrations of the feed nutrient, wherein the machine learning model comprises parameters defining or approximating a function, wherein the parameters are obtained by data fitting to experimental data comprising the concentrations of the feed nutrient in feed fed to animals and the concentrations of the biomarker in blood of at least a subset of the animals, wherein the machine learning model is trained on training data comprising independent variables and dependent variables;
obtain a nominal concentration of the feed nutrient in the feed of the at least one animal;
detect a deficiency in the nutritional state of the at least one animal by:

i) using the nominal concentration as input to the machine learning model to obtain as output an estimated concentration of the biomarker in the blood of the at least one animal and by determining a difference between the estimated concentration and the measured concentration of the biomarker; or
ii) using the measured concentration of the biomarker as input to the machine learning model to obtain an estimated concentration of the feed nutrient in the feed of the at least one animal and by determining a difference between the estimated concentration and the nominal concentration of the feed nutrient;

generate output data (182, 192) as a function of the difference to enable the deficiency in the nutritional state to be addressed on the basis of the output data.

14. The system according to claim 13, wherein the input interface subsystem (120) is further configured to access a nutritional log characterizing the feed fed to the at least one animal, and wherein the processor subsystem (160) is configured to determine the nominal concentration of the feed nutrient based on the nutritional log.

**15.** The system according to claim 14, wherein the nutritional log is indicative of a feed recipe of the feed, and wherein the processor subsystem (160) is configured to determine the nominal concentration of the feed nutrient using a database which maps feed recipes to feed nutrients and concentrations of the feed nutrients.

**16.** The system according to any one of claims 13 to 15, wherein the computer system (100) is configured to, if the difference exceeds a threshold, provide a sensory perceptible output signal (192) representing a recommendation to measure an actual concentration of the feed nutrient in the feed fed to the at least one animal.

**17.** The system according to any one of claims 13 to 16, further comprising an actuator interface (180) to an actuator (60) for adjusting a composition of nutritional supplements and/or for dispensing the nutritional supplements, wherein the processor subsystem (160) is configured to generate, as or as part of the output data, control data (182) for the actuator.

**18.** The system according to claim 17, wherein the nutritional supplements are dispensed to the at least one animal in separation of the feed, for example via drinking water (210) of the at least one animal or as additives to the feed.

**19.** The system according to claim 18, wherein the processor subsystem (160) is configured to control, via the actuator interface (180) and the actuator (60), a dispensing of the nutritional supplements into the drinking water of the at least one animal or to control preparation of an aqueous solution to be introduced into the drinking water.

**20.** The system according to claim 19, wherein the input interface subsystem (120) is further configured to access temperature data (42) which is indicative of a temperature in an environment of the at least one animal, and wherein the processor subsystem (160) is configured to, in the control of the actuator (60), account for a relation between the temperature and average water consumption of the at least one animal.

**21.** The system according to any one of claims 13 to 20, wherein the machine learning model configured to model the relationship between the concentrations of the biomarker and the concentrations of the feed nutrient as a further function of an age of a respective animal, wherein the processor subsystem is configured to access age data indicative of an average age of the at least one animal or an animal's individual age and use said age as input to the machine learning model.

**22.** The system according to any one of claims 13 to 21, wherein the computer system (100) is further configured to establish a graphical user interface, such as a web-accessible graphical user interface, wherein the graphical user interface is configured to allow uploading of the blood measurement data (22) or to establish a direct connection to the point-of-care device (20) to receive the blood measurement data, and to display the output data in the graphical user interface.

**23.** The system according to any one of claims 13 to 22, further comprising the point-of-care device (20) as a portable input device for the computer system (100).

**24.** The system according to any one of claims 13 to 23, wherein the computer system (100) is configured to communicate with the point-of-care device (20) or with a point-of-care data management system using an application programming interface (API).

**Patentansprüche**

**1.** Verfahren zur Futterüberwachung, umfassend:

Ex-vivo-Messen von Blutnährstoffkonzentrationen für eine Mehrzahl von Tieren;
Empfangen von Daten, die die Blutnährstoffkonzentrationen umfassen;
Empfangen eines Ernährungsprotokolls, das nominale Futternährstoffkonzentrationen in einem Futter der Mehrzahl von Tieren umfasst;
Bestimmen von abgeleiteten Futternährstoffkonzentrationen im Futter der Mehrzahl von Tieren basierend auf den Daten und unter Verwendung eines Modells für maschinelles Lernen, wobei die abgeleiteten Futternährstoffkonzentrationen mit Konzentrationen einer Mehrzahl von (Mikro-)Nährstoffbiomarkern assoziiert sind, die einen Ernährungszustand der Mehrzahl von Tieren angeben;
Vergleichen der abgeleiteten Futternährstoffkonzentrationen mit den entsprechenden nominalen Futter-

EP 4 312 582 B1

nährstoffkonzentrationen;

basierend auf dem Vergleich Bestimmen einer oder mehrerer Abweichungen zwischen den abgeleiteten Futternährstoffkonzentrationen und den entsprechenden nominalen Futternährstoffkonzentrationen; und

nach dem Bestimmen der einen oder der mehreren Abweichungen Ändern einer Futterzusammensetzung, um ein Problem zu beheben, das mit der einen oder den mehreren Abweichungen in dem Futter assoziiert ist.

2.  Verfahren nach Anspruch 1, wobei die Mehrzahl von (Mikro-)Nährstoffbiomarkern, die den Ernährungszustand angeben, Biomarker umfassen, die mit einem Ion, einem Vitamin, einem Carotinoid, einem Gesamtproteingehalt und Myo-Inositol assoziiert sind.

3.  Verfahren nach Anspruch 2, wobei das Ion ausgewählt ist aus der Gruppe bestehend aus Natrium, Kalium, Chlorid, Phosphor und Calcium, und wobei das Eisen kein Spurenmineral ist.

4.  Verfahren nach einem der Ansprüche 2 oder 3, wobei das Vitamin ausgewählt ist aus der Gruppe bestehend aus Vitamin A, Vitamin C, Vitamin D und Vitamin E.

5.  Verfahren nach einem der Ansprüche 1 bis 4, wobei Nährstoffe und Mikronährstoffe, deren Blutkonzentrationen aufgrund postabsorptiver Veränderungen, hormoneller und/oder transienter Reaktionen begrenzt sind, von der Mehrzahl von (Mikro-)Nährstoffbiomarkern ausgeschlossen sind und wobei Aminosäuren, Kohlenhydrate, Stärken, Fette und Lipide von der Mehrzahl von (Mikro-)Nährstoffbiomarkern ausgeschlossen sind.

6.  Verfahren nach einem der Ansprüche 1 bis 5, ferner umfassend:

    vor dem Ändern der Futterzusammensetzung Bestimmen der tatsächlichen Futternährstoffkonzentrationen im Futter unter Verwendung eines nasschemischen Verfahrens;

    Bestimmen zusätzlicher Abweichungen zwischen den tatsächlichen Futternährstoffkonzentrationen und den entsprechenden nominalen Futternährstoffkonzentrationen;

    nach dem Bestimmen der zusätzlichen Abweichungen Ändern der Futterzusammensetzung, um ein Problem zu beheben, das mit den zusätzlichen Abweichungen in dem Futter assoziiert ist; und

    nach dem Bestimmen, dass es keine Abweichung zwischen den tatsächlichen Futternährstoffkonzentrationen und den entsprechenden nominalen Futternährstoffkonzentrationen gibt, Empfehlen einer Maßnahme zur Erhöhung der Bioverfügbarkeit eines Nährstoffs bei der Mehrzahl von Tieren.

7.  Verfahren nach einem der Ansprüche 1 bis 6, ferner umfassend:
    Trainieren des Modells für maschinelles Lernen basierend auf historischen Daten, die Blutnährstoffkonzentrationen umfassen, die mit einem Satz von Tieren assoziiert sind, und einem historischen Ernährungsprotokoll für den Satz von Tieren.

8.  Verfahren nach einem der Ansprüche 1 bis 7, wobei die Mehrzahl von Tieren aus einer gleichen Gruppe stammt.

9.  Verfahren nach einem der Ansprüche 1 bis 8, wobei es sich bei der Mehrzahl von Tieren um monogastrische Tiere handelt, die einen Vogel oder ein Schwein umfassen, und es sich bei der Mehrzahl von Tieren am meisten bevorzugt um ein Huhn, einen Truthahn, eine Ente oder ein Gans handelt, und wobei das Huhn vorzugsweise ein Broiler ist.

10. Verfahren nach einem der Ansprüche 1 bis 9, wobei das Empfangen der Daten das Empfangen der Daten umfasst, die Blutnährstoffkonzentrationen umfassen, die von mindestens einer Point-of-Care-Vorrichtung gemessen wurden.

11. Verfahren nach einem der Ansprüche 1 bis 10, wobei die Mehrzahl von (Mikro-)Nährstoffbiomarkern eine Menge von mindestens einem (Mikro-)Nährstoff angibt, die ein Tier aufgenommen hat.

12. Verfahren zur Futterüberwachung, umfassend:

    Ex-vivo-Messen von Biomarker-Nährstoffkonzentrationen für eine Mehrzahl von Tieren;

    Empfangen von Daten, die die Biomarker-Nährstoffkonzentrationen umfassen;

    Empfangen eines Ernährungsprotokolls, das nominale Futternährstoffkonzentrationen in einem Futter der Mehrzahl von Tieren umfasst;

    Bestimmen von abgeleiteten Biomarker-Nährstoffkonzentrationen in der Mehrzahl von Tieren basierend auf dem Ernährungsprotokoll und unter Verwendung eines Modells für maschinelles Lernen, wobei die abgeleiteten

43

Biomarker-Nährstoffkonzentrationen mit nominalen Futternährstoffkonzentrationen in einem Futter der Mehrzahl von Tieren assoziiert sind;

Vergleichen der abgeleiteten Biomarker-Nährstoffkonzentrationen mit den entsprechenden gemessenen Biomarker-Nährstoffkonzentrationen;

basierend auf dem Vergleich Bestimmen einer oder mehrerer Abweichungen zwischen den abgeleiteten Biomarker-Nährstoffkonzentrationen und den entsprechenden gemessenen Biomarker-Nährstoffkonzentrationen; und

nach dem Bestimmen der einen oder der mehreren Abweichungen Ändern einer Futterzusammensetzung, um ein Problem zu beheben, das mit der einen oder den mehreren Abweichungen assoziiert ist.

**13.** System zum Erkennen eines Mangels in einem Ernährungszustand mindestens eines Tiers, wobei das System mindestens ein Computersystem (100) umfasst, das Folgendes umfasst:

mindestens eine Point-of-Care-Vorrichtung (20), die zur Ex-vivo-Messung einer Blutprobe ausgelegt ist, wobei die Blutmessdaten von mindestens einem Tier stammen;
und mindestens ein Computersystem (100), das Folgendes umfasst:

ein Eingabeschnittstellen-Subsystem (120), das dazu ausgelegt ist, auf Blutmessdaten (22) zuzugreifen, die durch mindestens eine Point-of-Care-Vorrichtung (20) erzeugt werden;
ein Prozessorsubsystem (160), das zu Folgendem ausgelegt ist:

Bestimmen einer gemessenen Konzentration eines Biomarkers eines Futternährstoffs im Futter des mindestens einen Tiers aus den Blutmessdaten;
Bereitstellen eines Modells für maschinelles Lernen (142), das dazu ausgelegt ist, eine Beziehung zwischen Konzentrationen des Biomarkers und Konzentrationen des Futternährstoffs zu modellieren, wobei das Modell für maschinelles Lernen Parameter umfasst, die eine Funktion definieren oder approximieren, wobei die Parameter durch Datenanpassung an experimentelle Daten erhalten werden, die die Konzentrationen des Futternährstoffs in Futter, das an Tiere verfüttert wird, und die Konzentrationen des Biomarkers in Blut mindestens einer Teilmenge der Tiere umfassen, wobei das Modell für maschinelles Lernen mit Trainingsdaten trainiert wird, die unabhängige Variablen und abhängige Variablen umfassen;
Erhalten einer nominalen Konzentration des Futtermittelnährstoffs im Futter des mindestens einen Tiers;
Erkennen eines Mangels im Ernährungszustand des mindestens einen Tiers durch:

i) Verwenden der nominalen Konzentration als Eingabe in das Modell für maschinelles Lernen, um als Ausgabe eine geschätzte Konzentration des Biomarkers im Blut des mindestens einen Tiers zu erhalten, und durch Bestimmen einer Differenz zwischen der geschätzten Konzentration und der gemessenen Konzentration des Biomarkers; oder
ii) Verwenden der gemessenen Konzentration des Biomarkers als Eingabe in das Modell für maschinelles Lernen, um eine geschätzte Konzentration des Futternährstoffs im Futter des mindestens einen Tiers zu erhalten, und durch Bestimmen einer Differenz zwischen der geschätzten Konzentration und der nominalen Konzentration des Futternährstoffs;

Erzeugen von Ausgabedaten (182, 192) als eine Funktion der Differenz, um zu ermöglichen, dass der Mangel in dem Ernährungszustand basierend auf den Ausgabedaten behoben wird.

**14.** System nach Anspruch 13, wobei das Eingabeschnittstellen-Subsystem (120) ferner dazu ausgelegt ist, auf ein Ernährungsprotokoll zuzugreifen, das das Futter beschreibt, das dem mindestens einen Tier verfüttert wird, und wobei das Prozessorsubsystem (160) dazu ausgelegt ist, die nominale Konzentration des Futternährstoffs basierend auf dem Ernährungsprotokoll zu bestimmen.

**15.** System nach Anspruch 14, wobei das Ernährungsprotokoll ein Futterrezept des Futters angibt und wobei das Prozessorsubsystem (160) dazu ausgelegt ist, die nominale Konzentration des Futternährstoffs unter Verwendung einer Datenbank zu bestimmen, die Futterrezepte Futternährstoffen und Konzentrationen der Futternährstoffe zuordnet.

**16.** System nach einem der Ansprüche 13 bis 15, wobei das Computersystem (100) so ausgelegt ist, dass es, wenn die Differenz eine Schwelle überschreitet, ein sensorisch wahrnehmbares Ausgangssignal (192) bereitstellt, das eine Empfehlung zur Messung einer tatsächlichen Konzentration des Futternährstoffs im Futter darstellt, das dem mindestens einen Tier verfüttert wird.

17. System nach einem der Ansprüche 13 bis 16, ferner umfassend eine Aktuator-Schnittstelle (180) zu einem Aktuator (60) zum Anpassen einer Zusammensetzung von Nahrungsergänzungsmitteln und/oder zum Abgeben der Nahrungsergänzungsmittel, wobei das Prozessorsubsystem (160) dazu ausgelegt ist, als Ausgabedaten oder als Teil davon Steuerdaten (182) für den Aktuator zu erzeugen.

18. System nach Anspruch 17, wobei die Nahrungsergänzungsmittel getrennt vom Futter, beispielsweise über Trinkwasser (210) des mindestens einen Tiers, oder als Zusatzstoffe zum Futter an das mindestens eine Tier abgegeben werden.

19. System nach Anspruch 18, wobei das Prozessorsubsystem (160) dazu ausgelegt ist, über die Aktuator-Schnittstelle (180) und den Aktuator (60) eine Abgabe der Nahrungsergänzungsmittel in das Trinkwasser des mindestens einen Tiers zu steuern oder die Zubereitung einer wässrigen Lösung, die in das Trinkwasser eingeführt werden soll, zu steuern.

20. System nach Anspruch 19, wobei das Eingabeschnittstellen-Subsystem (120) ferner dazu ausgelegt ist, auf Temperaturdaten (42) zuzugreifen, die eine Temperatur in einer Umgebung des mindestens einen Tiers angeben, und wobei das Prozessorsubsystem (160) dazu ausgelegt ist, bei der Steuerung des Aktuators (60) eine Beziehung zwischen der Temperatur und dem durchschnittlichen Wasserverbrauch des mindestens einen Tiers zu berücksichtigen.

21. System nach einem der Ansprüche 13 bis 20, wobei das Modell für maschinelles Lernen dazu ausgelegt ist, die Beziehung zwischen den Konzentrationen des Biomarkers und den Konzentrationen des Futternährstoffs als eine weitere Funktion eines Alters eines jeweiligen Tiers zu modellieren, wobei das Prozessorsubsystem dazu ausgelegt ist, auf Altersdaten zuzugreifen, die ein durchschnittliches Alter des mindestens einen Tiers oder das individuelle Alter eines Tiers angeben, und das Alter als Eingabe in das Modell für maschinelles Lernen zu verwenden.

22. System nach einem der Ansprüche 13 bis 21, wobei das Computersystem (100) ferner dazu ausgelegt ist, eine grafische Benutzeroberfläche, wie etwa eine über das Web aufrufbare grafische Benutzeroberfläche, einzurichten, wobei die grafische Benutzeroberfläche dazu ausgelegt ist, Hochladen der Blutmessdaten (22) zu ermöglichen oder eine direkte Verbindung mit der Point-of-Care-Vorrichtung (20) herzustellen, um die Blutmessdaten zu empfangen und die Ausgabedaten auf der grafischen Benutzeroberfläche anzuzeigen.

23. System nach einem der Ansprüche 13 bis 22, ferner umfassend die Point-of-Care-Vorrichtung (20) als tragbare Eingabevorrichtung für das Computersystem (100).

24. System nach einem der Ansprüche 13 bis 23, wobei das Computersystem (100) dazu ausgelegt ist, unter Verwendung einer Anwendungsprogrammierschnittstelle (API) mit der Point-of-Care-Vorrichtung (20) oder mit einem Point-of-Care-Datenverwaltungssystem zu kommunizieren.

**Revendications**

1. Procédé pour la surveillance d'une alimentation comprenant :

   la mesure ex vivo de concentrations de nutriments dans le sang pour une pluralité d'animaux
   la réception de données comprenant lesdites concentrations de nutriments dans le sang ;
   la réception d'un registre nutritionnel comprenant des concentrations nominales de nutriments alimentaires dans une alimentation de la pluralité d'animaux ;
   la détermination, sur la base des données et à l'aide d'un modèle d'apprentissage automatique, de concentrations déduites de nutriments alimentaires dans l'alimentation de la pluralité d'animaux, les concentrations déduites de nutriments alimentaires étant associées à des concentrations d'une pluralité de biomarqueurs de (micro)nutriments révélateurs d'un état nutritionnel de la pluralité d'animaux ;
   la comparaison des concentrations déduites de nutriments alimentaires avec les concentrations nominales correspondantes de nutriments alimentaires ;

sur la base de la comparaison, la détermination d'un ou plusieurs écarts entre les concentrations déduites de nutriments alimentaires et les concentrations nominales correspondantes de nutriments alimentaires ; et après la détermination du ou des écarts, la modification d'une composition d'alimentation pour remédier à un problème associé à l'écart ou aux écarts dans l'alimentation.

2. Procédé selon la revendication 1, dans lequel la pluralité de biomarqueurs de (micro)nutriments révélateurs de l'état nutritionnel comprennent des biomarqueurs associés à un ion, une vitamine, un caroténoïde, une teneur totale en protéines et le myo-inositol.

3. Procédé selon la revendication 2, dans lequel l'ion est choisi dans le groupe constitué par les ions sodium, potassium, chlorure, phosphore et calcium et dans lequel le fer n'est pas un oligoélément.

4. Procédé selon l'une quelconque des revendications 2 ou 3, dans lequel la vitamine est choisie dans le groupe constitué par la vitamine A, la vitamine C, la vitamine D et la vitamine E.

5. Procédé selon l'une quelconque des revendications 1 à 4, dans lequel les nutriments et les micronutriments dont les concentrations dans le sang sont plafonnées en raison de modifications post-absorptives, de réponses hormonales et/ou de réponses transitoires sont exclus de la pluralité de biomarqueurs de (micro)nutriments et dans lequel les acides aminés, les glucides, les amidons, les matières grasses et les lipides sont exclus de la pluralité de biomarqueurs de (micro)nutriments.

6. Procédé selon l'une quelconque des revendications 1 à 5, comprenant en outre :

avant la modification de la composition d'alimentation, la détermination de concentrations réelles de nutriments alimentaires dans l'alimentation à l'aide d'un procédé de chimie par voie humide ; la détermination d'écarts supplémentaires entre les concentrations réelles de nutriments alimentaires et les concentrations nominales correspondantes de nutriments alimentaires ; après la détermination des écarts supplémentaires, la modification de la composition d'alimentation pour remédier à un problème associé aux écarts supplémentaires dans l'alimentation ; et après la détermination de l'absence d'écart entre les concentrations réelles de nutriments alimentaires et les concentrations nominales correspondantes de nutriments alimentaires, la recommandation d'une mesure pour augmenter la biodisponibilité d'un nutriment chez la pluralité d'animaux.

7. Procédé selon l'une quelconque des revendications 1 à 6, comprenant en outre : l'entraînement, sur la base de données historiques comprenant des concentrations de nutriments dans le sang associées à un ensemble d'animaux et du registre nutritionnel historique pour l'ensemble d'animaux, du modèle d'apprentissage automatique.

8. Procédé selon l'une quelconque des revendications 1 à 7, dans lequel la pluralité d'animaux proviennent d'un même troupeau.

9. Procédé selon l'une quelconque des revendications 1 à 8, dans lequel la pluralité d'animaux sont des animaux monogastriques, comprenant un oiseau ou un porc, et la pluralité d'animaux sont le plus préférablement un poulet, une dinde, un canard ou une oie et dans lequel le poulet est de préférence un poulet de chair.

10. Procédé selon l'une quelconque des revendications 1 à 9, dans lequel la réception des données comprend la réception des données comprenant des concentrations de nutriments dans le sang qui ont été mesurées à partir d'au moins un dispositif au point de soins.

11. Procédé selon l'une quelconque des revendications 1 à 10, dans lequel la pluralité de biomarqueurs de (micro) nutriments sont révélateurs d'une quantité d'au moins un (micro)nutriment qu'un animal a ingéré.

12. Procédé pour la surveillance d'une alimentation comprenant :

la mesure ex vivo de concentrations de nutriments biomarqueurs pour une pluralité d'animaux la réception de données comprenant lesdites concentrations de nutriments biomarqueurs ; la réception d'un registre nutritionnel comprenant des concentrations nominales de nutriments alimentaires dans une alimentation de la pluralité d'animaux ;

**EP 4 312 582 B1**

la détermination, sur la base du registre nutritionnel et à l'aide d'un modèle d'apprentissage automatique, de concentrations déduites de nutriments biomarqueurs chez la pluralité d'animaux, les concentrations déduites de nutriments biomarqueurs étant associées à des concentrations nominales de nutriments alimentaires dans une alimentation de la pluralité d'animaux ;

la comparaison des concentrations déduites de nutriments biomarqueurs avec les concentrations mesurées correspondantes de nutriments alimentaires biomarqueurs ;

sur la base de la comparaison, la détermination d'un ou plusieurs écarts entre les concentrations déduites de nutriments biomarqueurs et les concentrations mesurées correspondantes de nutriments biomarqueurs ; et

après la détermination du ou des écarts, la modification d'une composition d'alimentation pour remédier à un problème associé à l'écart ou aux écarts.

**13.** Système de détection d'une carence d'un état nutritionnel d'au moins un animal, le système comprenant au moins un système informatique (100) comprenant :

au moins un dispositif au point de soins (20), conçu pour la mesure ex vivo d'un échantillon de sang, les données de mesure sanguine étant celles d'au moins un animal ;

et au moins un système informatique (100) comprenant :

un sous-système interface d'entrée (120) conçu pour accéder à des données de mesure sanguine (22) générées par au moins un dispositif au point de soins (20) ;

un sous-système processeur (160) conçu pour :

déterminer, à partir des données de mesure sanguine, une concentration mesurée d'un biomarqueur d'un nutriment alimentaire dans l'alimentation de l'au moins un animal ;

fournir un modèle d'apprentissage automatique (142) conçu pour modéliser une relation entre les concentrations du biomarqueur et les concentrations du nutriment alimentaire, le modèle d'apprentissage automatique comprenant des paramètres définissant ou approximant une fonction, les paramètres étant obtenus par l'ajustement de données à des données expérimentales comprenant les concentrations du nutriment alimentaire dans l'alimentation nourrissant les animaux et les concentrations du biomarqueur dans le sang d'au moins un sous-ensemble des animaux, le modèle d'apprentissage automatique étant entraîné sur des données d'entraînement comprenant des variables indépendantes et des variables dépendantes ;

obtenir une concentration nominale du nutriment alimentaire dans l'alimentation de l'au moins un animal ;

détecter une carence de l'état nutritionnel de l'au moins un animal par :

i) l'utilisation de la concentration nominale en tant qu'entrée dans le modèle d'apprentissage automatique pour obtenir en tant que sortie une concentration estimée du biomarqueur dans le sang de l'au moins un animal et la détermination d'une différence entre la concentration estimée et la concentration mesurée du biomarqueur ; ou

ii) l'utilisation de la concentration mesurée du biomarqueur en tant qu'entrée dans le modèle d'apprentissage automatique pour obtenir une concentration estimée du nutriment alimentaire dans l'alimentation de l'au moins un animal et la détermination d'une différence entre la concentration estimée et la concentration nominale du nutriment alimentaire ;

générer des données de sortie (182, 192) en fonction de la différence pour permettre de remédier à la carence de l'état nutritionnel sur la base des données de sortie.

**14.** Système selon la revendication 13, dans lequel le sous-système interface d'entrée (120) est en outre conçu pour accéder à un registre nutritionnel caractérisant l'alimentation nourrissant l'au moins un animal et dans lequel le sous-système processeur (160) est conçu pour déterminer la concentration nominale du nutriment alimentaire sur la base du registre nutritionnel.

**15.** Système selon la revendication 14, dans lequel le registre nutritionnel est révélateur d'une recette d'alimentation de l'alimentation et dans lequel le sous-système processeur (160) est conçu pour déterminer la concentration nominale du nutriment alimentaire à l'aide d'une base de données qui établit la correspondance entre les recettes d'alimentation et les nutriments alimentaires et les concentrations des nutriments alimentaires.

**16.** Système selon l'une quelconque des revendications 13 à 15, dans lequel le système informatique (100) est conçu pour, si la différence dépasse un seuil, fournir un signal de sortie sensoriel perceptible (192) représentant une recommandation pour mesurer une concentration réelle du nutriment alimentaire dans l'alimentation nourrissant l'au moins un animal.

**17.** Système selon l'une quelconque des revendications 13 à 16, comprenant en outre une interface d'actionneur (180) avec un actionneur (60) pour l'ajustement d'une composition de suppléments nutritionnels et/ou pour la distribution des suppléments nutritionnels, dans lequel le sous-système processeur (160) est conçu pour générer, en tant que données de sortie ou en tant que partie des données de sortie, des données de commande (182) pour l'actionneur.

**18.** Système selon la revendication 17, dans lequel les suppléments nutritionnels sont distribués à l'au moins un animal séparément de l'alimentation, par exemple par le biais de l'eau d'abreuvement (210) de l'au moins un animal ou en tant qu'additifs à l'alimentation.

**19.** Système selon la revendication 18, dans lequel le sous-système processeur (160) est conçu pour commander, par le biais de l'interface d'actionneur (180) et de l'actionneur (60), une distribution des suppléments nutritionnels dans l'eau d'abreuvement de l'au moins un animal ou pour commander la préparation d'une solution aqueuse à introduire dans l'eau d'abreuvement.

**20.** Système selon la revendication 19, dans lequel le sous-système interface d'entrée (120) est en outre conçu pour accéder à des données de température (42) qui sont révélatrices d'une température dans un environnement de l'au moins un animal et dans lequel le sous-système processeur (160) est conçu pour, dans la commande de l'actionneur (60), tenir compte d'une relation entre la température et la consommation moyenne d'eau de l'au moins un animal.

**21.** Système selon l'une quelconque des revendications 13 à 20, dans lequel le modèle d'apprentissage automatique est conçu pour modéliser la relation entre les concentrations du biomarqueur et les concentrations du nutriment alimentaire en fonction en outre d'un âge d'un animal respectif, dans lequel le sous-système processeur est conçu pour accéder à des données d'âge révélatrices d'un âge moyen de l'au moins un animal ou d'un âge individuel d'un animal et utiliser ledit âge en tant qu'entrée dans le modèle d'apprentissage automatique.

**22.** Système selon l'une quelconque des revendications 13 à 21, dans lequel le système informatique (100) est en outre conçu pour créer une interface utilisateur graphique, telle qu'une interface utilisateur graphique accessible par internet, l'interface utilisateur graphique étant conçue pour permettre le téléchargement des données de mesure sanguine (22) ou pour établir une connexion directe avec le dispositif au point de soins (20) pour recevoir les données de mesure sanguine et pour afficher les données de sortie dans l'interface utilisateur graphique.

**23.** Système selon l'une quelconque des revendications 13 à 22, comprenant en outre le dispositif au point de soins (20) en tant que dispositif d'entrée portatif pour le système informatique (100).

**24.** Système selon l'une quelconque des revendications 13 à 23, dans lequel le système informatique (100) est conçu pour communiquer avec le dispositif au point de soins (20) ou avec un système de gestion de données au point de soins à l'aide d'une interface de programmation d'application (API).

Fig. 1

Fig. 2

Fig. 3A

300

320

340

350

20

22

Fig. 3B

340

ADJ:
NUTR↑

Fig. 4A

Fig. 4B

$$y = -0.2806x^2 + 2.3095x + 10.453$$
$$R^2 = 0.5183$$

Fig 4C

Fig. 4D

**Sodium**

Fig 5.

```
PROVIDE PARAMETERIZED MODEL          ACCESS BLOOD MEASUREMENT DATA
                                                              410
                  430
                                     DETERMINE BIOMARKER
400                                  CONCENTRATION C_B
                                                              420

              OBTAIN NOMINAL CONCENTRATION OF FEED
              NUTRIENT C_{F,N}                        440

              DETECT NUTRITIONAL DEFICIENCY USING
              PARAMETERIZED MODEL                     450

  GENERATE         Y      D>Th?
  OUTPUT

       460
```

Fig. 6

PROVIDE
PARAMETER-
IZED MODEL

PROVIDE
BLOOD
MEASUREM.
DATA

PROVIDE
NUTRIENT
CONCENTR.
DATA

510

520

530

MODEL DATA FITTING

540

ADAPT PARAMETERS &
OUTPUT MODEL

550

500

Fig. 7

610

600

Fig. 8

Fig. 9

```
                           ┌──────────────┐
                           │  select flock │
                           └───────┬──────┘
                                   │
                           ┌───────▼──────────────────┐
                           │ determine nutrient requirement │
                           │  (nominal concentration)  │
                           └───────┬──────────────────┘
                                   │
                           ┌───────▼──────┐
                           │    provide    │
                           │  feed receipe │
                           └───────┬──────┘
                                   │
                           ┌───────▼──────┐
                           │  prepare feed │
                           └───────┬──────┘
```

feed flock
with prepared feed

stock sample of
prepared feed

determine blood nutrient
concentration ex vivo

Model:
is blood nutrient concentration
in range?

yes          no

prepare next batch of
feed in the same
manner

analyze feed sample

nominal concentration
=
actual concentration ?

yes          no

take measures to
increase
bioavailability

prepare next batch of feed
more carefully

and/or

modify current feed for
current flock

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2008234995 A
- US 2016012748 A1
- WO 2020109348 A1

**Non-patent literature cited in the description**

- **KNUDSEN, K.E.B.** Fiber and nonstarch polysaccharide content and variation in common crops used in broiler diets.. *Poult. Sci.*, 2014, vol. 93, 2380-2393
- **ODJO, S. et al.** Influence of drying and hydrothermal treatment of corn on the denaturation of salt-soluble proteins and color parameters. *J. Food Engineering.*, 2012, vol. 109, 561-570
- **YIN, D., YUAN et al.** Effect of storage time on the characteristics of corn and efficiency of its utilization in broiler chickens. *Anim. Nutr.*, 2017, vol. 3, 252-257
- **DEYHIM et al.** Effect of heat stress and drinking water salt supplements on plasma electrolytes and aldosterone concentration in broiler chickens. *Int J Biometeorol.*, May 1995, vol. 38 (4), 216-7
- Chronic heat stress and respiratory alkalosis: occurrence and treatment in broiler chicks. **TEETER R.G.** ; **SMITH M.O.** ; **OWENS F.N.** Proc. Georgia Nutr. Conf. for the Feed Indus. Univ. Georgia, 2000, 160-169
- **J. HAYAT** ; **D. BALNAVE** ; **J. BRAKE**. *British Poultry Science*, 1999, vol. 40 (3), 411-418